(19) Europäisches Patentamt
European Patent Office
Office européen des brevets

(11) Veröffentlichungsnummer: **0 106 800**
**A1**

(12) **EUROPÄISCHE PATENTANMELDUNG**

(21) Anmeldenummer: 83810449.5

(22) Anmeldetag: 04.10.83

(51) Int. Cl.³: C 07 D 307/79
C 07 D 405/04
//C07D307/83, C07D295/08,
C07D295/10, C07C49/84,
C07C93/14

(30) Priorität: 08.10.82 CH 5926/82
22.04.83 CH 2177/83

(43) Veröffentlichungstag der Anmeldung: 25.04.84 Patentblatt 84/17

(84) Benannte Vertragsstaaten: AT BE CH DE FR IT LI LU NL SE

(71) Anmelder: CIBA-GEIGY AG
Postfach
CH-4002 Basel(CH)

(72) Erfinder: Wenk, Paul, Dr.
Quellenweg 7
CH-4123 Allschwil(CH)

(72) Erfinder: Breitenstein, Werner, Dr.
St. Galler-Ring 179
CH-4054 Basel(CH)

(72) Erfinder: Baumann, Marcus, Dr.
Rührbergerstrasse 6
CH-4058 Basel(CH)

(54) **Furane.**

(57) Benzofurane bzw. 2,3-Dihydro-benzofurane der Formel

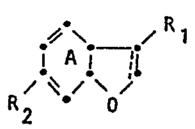

(I),

worin
$R_1$ für Wasserstoff oder einen aliphatischen Rest steht, $R_2$ eine durch einen zweiwertigen, gegebenenfalls durch mindestens ein Heteroatom unterbrochenen aliphatischen Rest disubstituierte Aminogruppe bedeutet und der aromatische Ring A zusätzlich substituiert sein kann, und ihre Salze weisen antiinflammatorische und/oder analgetische Aktivitäten auf.

EP 0 106 800 A1

CIBA-GEIGY AG
Basel (Schweiz)

4-14140/1+2/=

Furane

Die Erfindung betrifft neue Furane, insbesondere gegebenenfalls partiell hydrierte Benzofurane, und deren Salze, Verfahren zu ihrer Herstellung, solche Verbindungen enthaltende pharmazeutische Präparate, die Verwendung der erfindungsgemässen Verbindungen, z.B. als Arzneimittelwirkstoffe, sowie Verfahren zur Herstellung entsprechender pharmazeutischer Präparate.

Die Erfindung betrifft beispielsweise neue Benzofurane bzw. 2,3-Dihydro-benzofurane der Formel

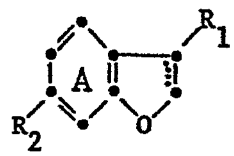

(I),

worin

$R_1$ für Wasserstoff oder einen aliphatischen Rest steht,

$R_2$ eine durch einen zweiwertigen, gegebenenfalls durch mindestens ein Heteroatom unterbrochenen aliphatischen Rest disubstituierte Aminogruppe bedeutet und der aromatische Ring

A zusätzlich substituiert sein kann und ihre Salze.

Die gestrichelte Linie der Formel I soll zum Ausdruck bringen, dass sowohl Benzofurane, d.h. im Furanteil ist eine Doppelbindung zwischen den Ringpositionen 2 und 3 lokalisiert, als auch 2,3-Dihydrobenzofurane, d.h. der Furanteil liegt in der 2,3-Dihydroform vor, umfasst sind.

Ein aliphatischer Rest $R_1$ ist in erster Linie Niederalkyl, ferner Niederalkenyl oder Niederalkinyl.

Die durch einen zweiwertigen, gegebenenfalls durch mindestens ein Heteroatom unterbrochenen aliphatischen Rest disubstituierte Aminogruppe $R_2$ bedeutet beispielsweise Niederalkylenamino oder Niederalkenylenamino mit einer oder zwei Doppelbindungen, jeweils z.B. mit 3 bis und mit 8, insbesondere 5 oder 6, Ringgliedern, wobei Niederalkylenamino zusätzlich ein oder zwei bzw. Niederalkenylenamino mit einer Doppelbindung ein ortho-anelliertes Benzosystem aufweisen kann. $R_2$ steht ebenso z.B. für Niederalkylenamino bzw. Niederalkenylenamino mit einer Doppelbindung, in welchem der Niederalkylen- bzw. Niederalkenylenteil jeweils durch mindestens ein Heteroatom, insbesondere Aza, Niederalkylaza, Oxa oder Thia, in erster Linie durch ein derartiges Heteroatom, unterbrochen ist, z.B. mit 3 bis und mit 8, insbesondere mit 5 oder 6, Ringgliedern. Niederalkylen bzw. Niederalkenylen ist im Zusammenhang mit $R_2$ verzweigt oder in erster Linie unverzweigt und weist z.B. 2 bis und mit 10, insbesondere 3 bis und mit 6, C-Atome auf. Derartige Reste sind beispielsweise Aziridin-1-yl, Azetidin-1-yl, Pyrrolidin-1-yl, 1-Piperidyl, Perhydroazepin-1-yl, Indolin-1-yl, Isoindolin-2-yl, Carbazol-9-yl, 2- oder 3-Pyrrolin-1-yl, Indol-1-yl, Pyrrol-1-yl, Imidazolidin-1-yl, Oxazolidin-3-yl, Thiazolidin-3-yl, Piperazin-1-yl, Morpholin-4-yl, Thiomorpholin-4-yl, 2-Imidazolin-1-yl, 4-Oxazolin-3-yl, 4-Thiazolin-3-yl.

Der aromatische Ring A kann zusätzlich, z.B. durch einen aliphatischen Rest, wie Niederalkyl, Hydroxyniederalkyl, Halogenniederalkyl, Niederalkenyl oder 2 benachbarte C-Atome überbrückendes Alkylen, wie Niederalkylen, z.B. mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro, ein- oder mehrfach substituiert oder, bis auf $R_2$, unsubstituiert sein.

Salze der erfindungsgemässen Verbindungen mit salzbildenden Gruppen sind vorzugsweise pharmazeutisch verwendbare Salze, wie pharmazeutisch verwendbare Additionssalze. Diese werden beispielsweise mit starken anorganischen Säuren, wie Mineralsäuren, z.B. Halogenwasserstoffsäuren, mit starken organischen Carbonsäuren, wie Niederalkancarbonsäuren, gegebenenfalls ungesättigte Dicarbonsäuren oder Hydroxycarbonsäuren, oder mit Sulfonsäuren, wie Niederalkan- oder gegebenenfalls substituierte Benzolsulfonsäuren, gebildet. Derartige Säureadditionssalze werden in der Regel nur mit solchen Verbindungen der Formel I gebildet, die eine basische Aminogruppe $R_2$ aufweisen. Umfasst sind ferner für pharmazeutische Verwendungen ungeeignete Salze, da letztere beispielsweise für die Isolierung bzw. Reinigung freier erfindungsgemässer Verbindungen sowie derer pharmazeutisch verwendbarer Salze verwendet werden können.

Die vor- und nachstehend verwendeten Allgemeindefinitionen haben, sofern nicht abweichend definiert, in erster Linie die folgenden Bedeutungen.

Der Ausdruck "nieder" bedeutet, dass entsprechend bezeichnete organische Gruppen oder Verbindungen vorzugsweise bis und mit 7, vor allem bis und mit 4, Kohlenstoffatome enthalten.

Niederalkyl ist z.B. Methyl, Ethyl, n-Propyl, Isopropyl, n-Butyl, Isobutyl, sek.-Butyl oder tert.-Butyl und umfasst ferner entsprechende Pentyl-, Hexyl- oder Heptylreste, während Niederalkenyl z.B. Vinyl, Allyl oder Methallyl und Niederalkinyl z.B. Propargyl bedeutet.

Niederalkylen als Substituent der Aminogruppe $R_2$ ist z.B. Ethylen, 1,3-Propylen, 1,4-Butylen, 2,3-Dimethyl-1,4-butylen, 1,5-Pentylen oder 2,5-Hexylen, während entsprechendes Niederalkenylen z.B. 1,4-But-1-enylen oder 1,4-But-2-enylen darstellt und Niederalkadienylen z.B. 1,4-Buta-1,3-dienylen ist.

Hydroxyniederalkyl ist z.B. Hydroxymethyl oder 2-Hydroxyethyl.

Halogenniederalkyl ist z.B. Chlormethyl, Trifluormethyl, oder 1,1,2-Trifluor-2-chlorethyl.

Zwei benachbarte C-Atome überbrückendes Niederalkylen mit 3 oder 4 Kettengliedern weist z.B. 3 bis und mit 10, insbesondere 3 bis und mit 8, in erster Linie 3 oder 4, C-Atome auf und bedeutet z.B. 1,3-Propylen oder 1,4-Butylen.

Niederalkoxy ist z.B. Methoxy, Ethoxy, n-Propyloxy, Isopropyloxy, n-Butyloxy, Isobutyloxy, sek.-Butyloxy oder tert.-Butyloxy.

Niederalkylthio ist z.B. Methyl-, Ethyl-, n-Propyl-, Isopropyl-, n-Butyl-, Isobutyl-, sek.-Butyl- oder tert.-Butylthio.

Niederalkansulfinyl bzw. -sulfonyl ist z.B. Methan-, Ethan-, n-Propan- oder Isopropansulfinyl bzw. -sulfonyl.

Halogen ist z.B. Halogen der Atomnummer bis und mit 35, wie Fluor, Chlor oder Brom, und umfasst ferner Iod.

Niederalkanoyloxy ist z.B. Acetyloxy, Propionyloxy, Butyryloxy, Isobutyryloxy oder Pivaloyloxy, während Niederalkanoyl z.B. Formyl, Acetyl, Propionyl, Butyryl, Isobutyryl oder Pivaloyl bedeutet.

Die erfindungsgemässen Verbindungen weisen wertvolle pharmakologische Eigenschaften auf. Insbesondere besitzen sie eine ausgeprägte antiinflammatorische Wirkung, die sich z.B. durch Reduktion des durch Carrageenin erzeugten Pfotenödems bei der Ratte ab einer Dosis von etwa 0,1 mg/kg p.o. analog der von Pasquale et al, Agents and Actions, 5, 256, (1975), beschriebenen Methode sowie im Adjuvans-Arthritis-Modell an der Ratte ab einer Dosis von etwa 1 mg/kg p.o. analog L. Risterer et al., Pharmacology, 2, 288 (1969), nachweisen lässt. Ausserdem hemmen Verbindungen der Formel I in vitro ab einer Konzentration von etwa 10 µ mol/l die Prostaglandinsynthese aus Arachidonsäure analog der von H.L. White et al, Prostaglandins, 7, 123 (1974), beschriebenen Methode.

Weiterhin weisen die erfindungsgemässen Verbindungen eine deutliche antinociceptive Wirkungskomponente auf, die sich z.B. aus der von L.C. Hendershot et al., J. Pharmacol. exp. Therap. 125, 237 (1959), beschriebenen Reduktion des durch Phenyl-p-benzochinon induzierten Writhing-Syndroms an der Maus ab einer Dosis von etwa 0,1 mg/kg p.o. ableiten lässt.

Infolge dessen lassen sich die erfindungsgemässen Wirksubstanzen als Antiinflammatorika und/oder (periphere) Analgetika verwenden.

Die Erfindung betrifft insbesondere Verbindungen der Formel I, worin $R_1$ für Wasserstoff, Niederalkyl, Niederalkenyl oder Niederalkinyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils im Niederalkylen- bzw. Niederalkenylenteil durch mindestens ein Aza, Niederalkylaza, Oxa oder Thia unterbrochenes Niederalkylenamino bzw. Niederalkenylenamino mit einer Doppelbindung, z.B. in allen Fällen mit 3 bis und mit 8 Ringgliedern und 2 bis und mit

10 C-Atomen, bedeutet, und der aromatische Ring A zusätzlich durch Niederalkyl, Hydroxyniederalkyl, Halogenniederalkyl, Niederalkenyl, zwei benachbarte C-Atome überbrückendes Alkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze.

Die Erfindung betrifft insbesondere Verbindungen der Formel I, worin $R_1$ für Wasserstoff oder Niederalkyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils mit 3 bis und mit 7 Ringgliedern, jeweils im Niederalkylen- bzw. Niederalkenylenteil durch ein Aza, Niederalkylaza, Oxa oder Thia unterbrochenes Niederalkylenamino mit 5 oder 6 Ringgliedern bzw. Niederalkenylenamino mit einer Doppelbindung und 5 Ringgliedern bedeutet und der aromatische Ring A zusätzlich durch Niederalkyl, Hydroxyniederalkyl, Halogenniederalkyl, Niederalkenyl, zwei benachbarte C-Atome überbrückendes Niederalkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze.

Die Erfindung betrifft insbesondere Verbindungen der Formel

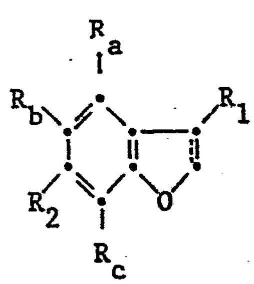

(Ia),

worin $R_1$ Wasserstoff oder Niederalkyl bedeutet, $R_2$ jeweils 5- bis 8-gliedriges Niederalkylenamino, Niederalkenylenamino, Azaniederalkylenamino, N'-Niederalkylaza-niederalkylenamino, Azaniederalkenylenamino, N'-Niederalkylaza-niederalkenylenamino, Oxa- bzw. Thia-niederalkylenamino, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, Hydroxyniederalkyl, Halogenniederalkyl, Niederalkenyl, Niederalkoxy, Niederalkylthio, Niederalkansulfinyl, Niederalkansulfonyl, Hydroxy, Halogen, Niederalkanoyloxy, Niederalkanoyl oder Nitro bedeuten oder $R_a$ gemeinsam mit $R_b$ 3- oder 4-gliedriges Alkylen darstellen und $R_c$ die vorstehend für $R_c$ angegebenen Bedeutungen hat, und ihre Salze.

Die Erfindung betrifft in erster Linie Verbindungen der Formel Ia, worin $R_1$ Wasserstoff oder Niederalkyl, z.B. mit bis und mit 4 C-Atomen, wie Methyl, bedeutet, $R_2$ Niederalkylenamino mit 5 oder 6 Ringgliedern und 4 bis und mit 10 C-Atomen, z.B. Pyrrolidin-1-yl oder 1-Piperidyl, Niederalkenylenamino mit einer oder zwei Doppelbindungen und 5 oder 6 Ringgliedern und 4 bis und mit 10 C-Atomen, wie 2- oder 3-Pyrrolin-1-yl oder Pyrrol-1-yl, oder 4-Oxaniederalkylenamino mit 6 Ringgliedern und 4 bis und mit 10 C-Atomen, z.B. Morpholin-4-yl, darstellt und $R_a$ und $R_c$ jeweils Wasserstoff bedeuten und $R_b$ Wasserstoff, Niederalkyl, Hydroxyniederalkyl, Halogenniederalkyl, Niederalkoxy, Niederalkylthio, z.B. jeweils mit bis und mit 4 C-Atomen, wie Methyl, Hydroxymethyl, Trifluormethyl, Methoxy oder Methylthio, Hydroxy, Halogen, z.B. der Atomnummer bis und mit 35, wie Chlor, oder Niederalkanoyloxy, z.B. mit 2 bis und mit 5 C-Atomen, wie Pivaloyloxy, darstellt oder $R_a$ und $R_b$ gemeinsam Niederalkylen mit 3 oder 4 Kettengliedern, z.B. mit 3 oder 4 C-Atomen, wie 1,4-Butylen, darstellen und $R_c$ Wasserstoff ist, und ihre Salze.

Die Erfindung betrifft in erster Linie Verbindungen der Formel Ia, worin $R_1$ Wasserstoff oder Niederalkyl mit bis und mit 4 C-Atomen, wie Methyl, bedeutet, $R_2$ 1-Pyrrolyl, 4-Morpholinyl, 3-Pyrrolin-1-yl oder unverzweigtes 4- bis 6-gliedriges Alkylenamino darstellt, $R_a$

und $R_c$ jeweils Wasserstoff bedeuten und $R_b$ Wasserstoff, Niederalkyl mit bis und mit 4 C-Atomen oder Halogen bis und mit Atomnummer 35, wie Methyl oder Chlor, darstellt oder $R_c$ Wasserstoff bedeutet und $R_a$ und $R_b$ gemeinsam 3 bis 4-gliedriges Alkylen oder einer der Reste $R_a$ und $R_b$ Halogen bis und mit Atomnummer 35 und der andere Niederalkyl mit bis 4 C-Atomen, wie Chlor oder Methyl, darstellen, und ihre Salze.

Die Erfindung betrifft in aller erster Linie einerseits Verbindungen der Formel

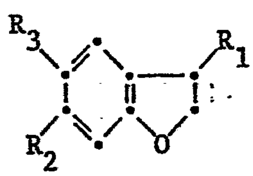

(Ib),

oder andererseits Verbindungen der Formel

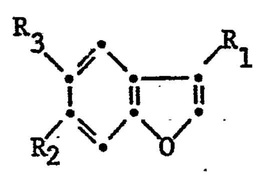

(Ic),

worin jeweils $R_1$ Wasserstoff oder Niederalkyl mit bis und mit 4 C-Atomen, wie Methyl, bedeutet, $R_2$ Pyrrolidin-1-yl, 1-Piperidyl, Pyrrol-1-yl oder Morpholin-4-yl darstellt und $R_3$ Wasserstoff, Niederalkyl mit bis und mit 4 C-Atomen, wie Methyl, oder Halogen der Atomnummer bis und mit 35, wie Chlor, bedeutet, und ihre Salze.

Die Erfindung betrifft in aller erster Linie Verbindungen der Formel Ic, worin $R_1$ Wasserstoff oder Niederalkyl mit bis und mit 4 C-Atomen, insbesondere Methyl, bedeutet, $R_2$ Pyrrol-1-yl darstellt und $R_3$ Niederalkyl mit bis und mit 4 C-Atomen, insbesondere Methyl, bedeutet.

Die Erfindung betrifft insbesondere die in den Beispielen genannten neuen Verbindungen und ihre Salze.

Die erfindungsgemässen Verbindungen und Salze von solchen Verbindungen mit salzbildenden Eigenschaften können, z.B. in an sich bekannter Weise, hergestellt werden, indem man beispielsweise

(a) in einer Verbindung der Formel

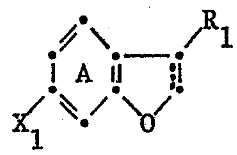

(II),

worin $X_1$ einen in $R_2$ überführbaren Rest bzw. durch $R_2$ ersetzbaren Rest bedeutet, $X_1$ in $R_2$ überführt bzw. durch $R_2$ ersetzt, oder,

(b) zur Herstellung von 2,3-Dihydrobenzofuranen der Formel I, in einer Verbindung der Formel

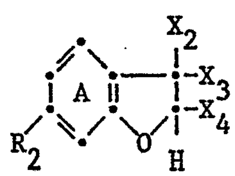

(III)

worin $X_2$ $R_1$ ist, einer der Reste $X_3$ und $X_4$ für einen durch Wasserstoff ersetzbaren Rest steht und der andere Wasserstoff ist bzw. $X_3$ und $X_4$ gemeinsam eine zusätzliche Bindung darstellen, $X_3$ oder $X_4$ durch Wasserstoff ersetzt bzw. die zusätzliche, durch $X_3$ und $X_4$ dargestellte Bindung reduziert, oder, wenn $R_1$ Wasserstoff ist, $X_2$ und $X_3$ gemeinsam für eine zweiwertige, durch 2 Wasserstoffatome ersetzbare Gruppe stehen und $X_4$ Wasserstoff ist, $X_2$ und $X_3$ jeweils durch 1 Wasserstoffatom ersetzt, oder,

(c) zur Herstellung von Benzofuranen bzw. 2,3-Dihydrobenzofuranen der Formel I, worin $R_1$ für einen aliphatischen Rest steht, in eine Verbindung der Formel

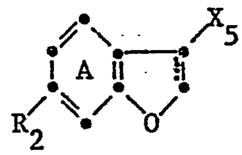

(IVa)

durch Umsetzung mit einer Verbindung der Formel $R_1-X_5'$ (IVb), worin einer der Reste $X_5$ und $X_5'$ gegebenenfalls reaktionsfähiges verestertes oder verethertes Hydroxy bedeutet und der andere für einen metallischen Rest steht oder worin $X_5$ und $X_5'$ jeweils Halogen bedeuten, und unter Abspaltung von $X_5-X_5'$ den Rest $R_1$ einführt, oder

(d) eine Verbindung der Formel

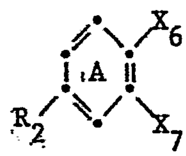

(V),

worin $X_6$ für die Gruppe der Formel $-CH(R_1)-CH_2-X_6'$ steht, wobei $X_6'$ für einen abspaltbaren Rest steht, und $X_7$ Hydroxy ist, oder worin, falls $R_1$ der Formel I für einen aliphatischen Rest steht, $X_6$ die Diazoniumgruppe oder Halogen bedeutet und $X_7$ für die Gruppe der Formel $-O-CH_2-CH = R_1'$ steht, wobei $R_1'$ ein dem Rest $R_1$ entsprechender, doppelt gebundener aliphatischer Rest ist, zu einer 2,3-Dihydrobenzofuranverbindung der Formel I cyclisiert bzw. eine Verbindung der Formel V, worin $X_6$ Wasserstoff ist und $X_7$ die Gruppe der Formel $-O-CH_2-C(=O)-R_1$ oder eine derivatisierte Form davon steht, oder worin $X_6$ die Gruppe der Formel $-C(R_1)=CH-X_6''$ darstellt, wobei $X_6''$ für einen abspaltbaren Rest steht, und $X_7$ Hydroxy ist, zu einer Benzofuranverbindung der Formel I cyclisiert, oder,

(e) zur Herstellung von Benzofuranen der Formel I, in einer Verbindung der Formel

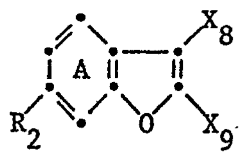

(VI),

worin einer der Reste $X_8$ und $X_9$ für einen durch Wasserstoff ersetzbaren Rest steht und der andere Wasserstoff oder, im Fall von $X_8$, ein von Wasserstoff verschiedener Rest $R_1$ bedeutet, $X_8$ bzw. $X_9$ durch Wasserstoff ersetzt, oder,

(f) zur Herstellung von Benzofuranen der Formel I, aus einer der Verbindung der Formel

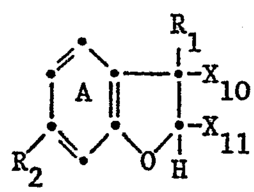

(VII),

worin einer der Reste $X_{10}$ und $X_{11}$ für einen abspaltbaren Rest steht und der andere Wasserstoff ist, oder worin $X_{10}$ und $X_{11}$ jeweils Halogen, Carboxy oder Wasserstoff sind, unter Bildung einer zusätzlichen Bindung, eine Verbindung $X_{10} - X_{11}$ abspaltet, oder,

(g) zur Herstellung von Benzofuranen der Formel I, worin $R_1$ für einen aliphatischen Rest steht, eine Verbindung der Formel

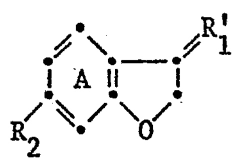

(VIII),

worin $R_1'$ ein dem Rest $R_1$ entsprechender, von Wasserstoff verschiedener, doppelt gebundener aliphatischer Rest darstellt, isomerisiert und, wenn erwünscht, eine erhaltene erfindungsgemässe Verbindung in eine andere erfindungsgemässe Verbindung überführt und/oder gewünschtenfalls ein erhaltenes Salz in die freie Verbindung überführt und/oder gewünschtenfalls eine erhaltene erfindungsgemässe Verbindung

mit salzbildenden Eigenschaften in ein Salz überführt und/oder gewünschtenfalls ein erhaltenes Isomerengemisch in die einzelnen Isomeren auftrennt.

Die Erfindung betrifft ebenfalls die nach den vorstehend beschriebenen Verfahren erhältlichen Verbindungen.

Die vor- und nachstehend in den Varianten a)-g) beschriebenen Umsetzungen werden in an sich bekannter Weise durchgeführt, sofern nicht abweichend aufgeführt, in Ab- oder üblicherweise in Anwesenheit eines geeigneten Lösungs- oder Verdünnungsmittel oder eines Gemisches derselben, wobei man unter Kühlen, bei Raumtemperatur oder unter Erwärmen, z.B. in einem Temperaturbereich von etwa -10°C bis etwa 250°C, vorzugsweise von etwa 40°C bis etwa 150°C, und, falls erforderlich, in einem geschlossenen Gefäss, gegebenenfalls unter Druck, in einer Inertgasatmosphäre und/oder unter wasserfreien Bedingungen arbeitet.

Das vor- und nachstehend aufgeführte Ausgangsmaterial der Formeln II, III, IVa, IVb, V, VI, VII und VIII kann ebenso nach an sich bekannten Methoden hergestellt werden.

Variante (a):

Ein in $R_2$ überführbarer Rest $X_1$ steht beispielsweise für die Aminogruppe oder die Gruppe der Formel -NH-alk-$Z_1$, in der alk ein dem Rest $R_2$ entsprechender, zweiwertiger, gegebenenfalls durch mindestens ein Heteroatom unterbrochener aliphatischer Rest und $Z_1$ gegebenenfalls reaktionsfähiges, insbesondere z.B. mit einer Mineralsäure oder Sulfonsäure, ferner Carbonsäure, verestertes Hydroxy bedeuten. $Z_1$ kann dementsprechend beispielsweise Halogen, wie Chlor oder Brom, Sulfato oder gegebenenfalls substituiertes Phenyl- bzw. Niederalkan-sulfonyloxy, wie p-Toluol-, p-Bromphenyl-, Methan- oder Trifluormethan-sulfonyloxy, ferner gegebenenfalls substituiertes Niederalkanoyloxy, wie Acetyl- oder Trifluoracetyloxy, darstellen.

Die Ueberführung von $X_1$ in $R_2$ erfolgt üblicher Weise in Gegenwart eines Kondensationsmittels, wie einer Säure oder insbesondere einer Base. Als Säuren kommen beispielsweise starke Protonsäuren, wie Mineralsäuren, z.B. Schwefel- oder eine Phosphorsäure, oder Sulfonsäuren, z.B. p-Toluolsulfonsäure, in Frage, während als geeignete Basen beispielswiese Alkalimetallhydroxide, z.B. Natronlauge, Alkalimetallniederalkanolate, wie Natriummethylat, gegebenenfalls cyclische tertiäre Stickstoffbasen, z.B. Triethylamin, Ethyldiisopropylamin, Piperidin, Collidin, 1,5-Diaza-bicyclo[4.3.0]non-5-en (DBN) oder 1,8-Diazabicyclo[5.4.0]undec-7-en (DBU), ferner Lithiumdiniederalkylamide, z.B. Lithium-diethyl- oder Lithium-diisopropyl-amid, verwendet werden.

In einer weiteren Ausführungsform zur Herstellung von Verbindungen der Formel I, worin $R_2$ Morpholin-4-yl bzw. Thiomorpholin-4-yl bedeutet, geht man beispielsweise vom Ausgangsmaterial der Formel II aus, worin $X_1$ für die Gruppe der Formel $-N(CH_2-CH_2-OH)_2$ bzw. $-N(CH_2-CH_2-SH)_2$ steht, und kondensiert dieses beispielsweise in Gegenwart einer starken Protonsäure, wie Schwefelsäure.
Ebenso kann man zu Verbindungen der Formel I gelangen, worin $R_2$ Morpholin-4-yl bzw. Thiomorpholin-4-yl bedeutet, indem man Ausgangsmaterial der Formel II, worin $X_1$ Amino ist, z.B. mit einem 2,2'-Dihalogen-, wie 2,2'-Dibrom-diethyl(thio)ether in Gegenwart eines basischen Kondensationsmittels, z.B. einem der vorstehend genannten, wie N-Ethyl-diisopropylamin, umsetzt. Dabei wird intermediär eine Verbindung der Formel II gebildet, wobei $X_1$ für die Gruppe der Formel $-NH-C_2H_4-O-C_2H_4-Hal$ bzw. die entsprechende Thioform steht. Zweckmässig werden derartige Ausgangsverbindungen nicht isoliert, sondern unter entsprechenden Reaktionsbedingungen weiterkondensiert.

Zur Herstellung von Verbindungen der Formel I, worin $R_2$ Pyrrol-1-yl bedeutet, geht man in erster Linie von Ausgangsverbindungen der Formel II aus, worin $X_1$ Amino bedeutet, und setzt diese z.B. mit 2,5-Diniederalkoxy-, wie 2,5-Dimethoxy-tetrahydrofuran, einem 4-Niederalkoxy-, wie 4-Methoxy-but-3-en-1-in oder dem gegebenenfalls

acetalisierten Succindialdehyd bzw. dessen tautomeren Enolform um.

Bedeutet $X_1$ z.B. Halogen, wie Chlor, Brom oder Iod, und ist in ortho-Stellung zu $X_1$ mindestens ein Substituent mit starkem -I- oder -M-Effekt, wie Halogen, Nitro oder Trifluormethyl, lokalisiert, kann $X_1$ durch $R_2$ ersetzt werden. Der Ersatz kann z.B. durch Umsetzung mit einer Verbindung der Formel $R_2$-H bzw. $R_2$-Me erfolgen, wobei $R_2$ für eine basische, durch einen zweiwertigen, gegebenenfalls durch mindestens ein Heteroatom unterbrochenen aliphatischen Rest disubstituierte Aminogruppe und Me für einen metallischen Rest, wie Alkalimetall, z.B. Lithium, stehen. In einzelnen Fällen ist es bei diesen Umsetzungen von Vorteil, unter Druck und/oder bei erhöhten Temperaturen zu arbeiten. Vorteilhaft setzt man die Amine im Ueberschuss ein.

Zur Herstellung von Benzofuranen der Formel II, worin $X_1$ für die Aminogruppe oder die Gruppe der Formel -NH-alk-$Z_1$ steht, geht man beispielsweise von einem 3-Nitrophenol aus und setzt dieses z.B. mit einer Verbindung der Formel $R_1$-CO-$CH_2$-Hal um, worin Hal für Halogen steht, bzw. einem entsprechenden Acetal davon, z.B. Chloraceton oder 2-Chlor-1,1-diethoxy-ethan, in Gegenwart einer Base, z.B. Kaliumcarbonat, und unter Erwärmen zu einer Verbindung der Formel

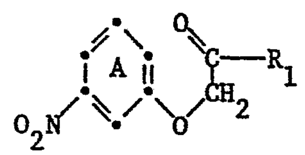

(IIa)

oder einem Acetal davon um. Nach Reduktion der Nitrogruppe, z.B. durch Hydrierung in Gegenwart eines Hydrierungskatalysators, wie Raney-Nickel, kann die resultierende Aminoverbindung z.B. mittels eines sauren Kondensationsmittels, wie konzentrierter Schwefelsäure, zur entsprechenden Verbindung der Formel II cyclisiert werden, worin $X_1$ Amino bedeutet, die gewünschtenfalls, z.B. durch Umsetzung mit höchstens einem Mol einer Verbindung der Formel $Z_1$-alk-$Z_1$, in eine Verbindung der Formel II übergeführt werden kann, worin $X_1$ für die Gruppe der

Formel -NH-alk-$Z_1$ steht. Die Herstellung von Ausgangsverbindungen der Formel II, worin $X_1$ Halogen bedeutet, kann analog, ausgehend z.B. von einem 3-Halogenphenol, über die Kondensation mit einer Verbindung der Formel $R_1$-CO-$CH_2$-Hal bzw. einem Acetal davon und die Cyclisierung erfolgen.

2,3-Dihydrobenzofurane der Formel II sind beispielsweise zugänglich, indem man z.B. ein 3-Halogen-1-niederalkoxy-benzol-Derivat in Gegenwart einer Lewissäure, z.B. Aluminiumchlorid, mit einem Acetylhalogenid acyliert. Dabei kann gleichzeitig der Niederalkylether zum entsprechenden Phenol gespalten werden. Dieses wird zweckmässig in üblicher Weise wieder verethert. Im nächsten Reaktionsschritt wird z.B. das Acetophenonderivat analog der Willgerodt- bzw. Kindler-Reaktion, z.B. mit Schwefel und Morpholin, umgesetzt, und das z.B. resultierende Thiomorpholid durch Säurebehandlung in die Verbindung der Formel

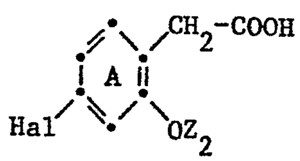

(IIb),

worin $Z_2$ Niederalkyl ist, übergeführt. Durch Umsetzung mit einem Halogenid der Formel $R_1$-Hal in Gegenwart einer starken Base, z.B. Natriumamid, kann ein von Wasserstoff verschiedener Rest $R_1$ eingeführt werden. Die nachfolgende Reduktion der Carboxygruppe mit Hilfe eines Hydrid-übertragenden Reagenz, z.B. Lithiumaluminiumhydrid, führt z.B. zu Verbindungen der Formel

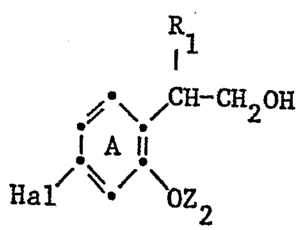

(IIc).

Durch Umsetzung mit einer Lewissäure, z.B. Bortribromid, können nach Massgabe entsprechender Konzentrationsverhältnisse der Ether gespalten und die Hydroxygruppe durch das entsprechende Halogenatom substituiert werden. Im nächsten Reaktionsschritt erfolgt durch Kondensation mit Hilfe einer Base, wie 1,8-Diazabicyclo[5.4.0]-undec-7-en, die Cyclisierung zu 2,3-Diyhdrobenzofuranen der Formel

II, worin $X_1$ Halogen bedeutet. Zur Herstellung entsprechender Aminoverbindungen der Formel II ($X_1$ = Amino) geht man z.B. von 1-Niederalkoxy-3-nitrobenzol-Verbindungen aus und führt die Reaktionssequenz in analoger Weise durch, wobei in derem Verlauf die Nitro- zur Aminogruppe reduziert und diese wiederum gegebenenfalls, z.B. durch Acylierung, vorübergehend geschützt werden kann.

Variante (b):

Ein durch Wasserstoff ersetzbarer Rest $X_3$ bzw. $X_4$ stellt beispielsweise Hydroxy, verestertes, z.B. mit einer Mineralsäure, Sulfonsäure oder organischen Carbonsäure verestertes, Hydroxy, verethertes Hydroxy, substituiertes Thio bzw. Seleno oder disubstituiertes Amino dar. Als Beispiele für derartige Reste seien genannt: Halogen, wie Chlor oder Brom, gegebenenfalls substituiertes Phenyl- bzw. Niederalkansulfonyloxy, wie p-Toluol-, p-Bromphenyl-, Methan- oder Trifluormethansulfonyloxy, Niederalkoxy, gegebenenfalls substituiertes Phenyl- bzw. Niederalkylthio bzw. -seleno, wie Phenyl- oder Methylthio bzw. -seleno, Diniederalkylamino, wie Dimethylamino, Di-sulfonyloxy-amino, wie Di-toluolsulfonyloxy-amino, oder Niederalkanoyloxy, wie Acetyloxy.

Der reduktive Austausch von $X_3$ bzw. $X_4$ durch Wasserstoff erfolgt in an sich bekannter Weise, z.B. durch Hydrierung in Gegenwart eines Hydrierungskatalysators, durch Reduktion mit einem Hydrid-übertragenden Reagenz oder durch ein metallisches Reduktionssystem aus Metall und protonenabspaltendem Mittel.

Als Hydrierungskatalysatoren seien beispielsweise Elemente der VIII. Nebengruppe des Periodensystems der Elemente oder deren Derivate, wie Palladium, Platin, Platinoxid, Cobalt, Raney-Nickel, Rhodium oder Tris-(triphenylphosphin)rhodium(I)halogenid, z.B. -chlorid, genannt, die gegebenenfalls auf einem geeigneten Trägermaterial, wie Aktivkohle, Erdalkalimetallcarbonat bzw. -sulfat oder einem Kieselgel, aufgezogen sind. Zu den Hydrid- übertragenden Reagenzien zählen beispielsweise geeignete Alkalimetallaluminiumhydride bzw. -borhydride oder Zinnhydride, wie Lithiumaluminiumhydrid, Natriumborhydrid, Lithiumtriethylborhydrid, Natriumcyanoborhydrid oder Triethylzinnhydrid. Der Metallbestandteil des metallischen Reduktionssystems ist beispielsweise ein unedles Metall, wie Alkali- oder Erdalkalimetall, z.B. Natrium, Lithium, Magnesium, Calcium, oder Uebergangsmetall, z.B. Zink, Zinn, Eisen, Titan, während als protonenabspaltendes Mittel z.B. Protonensäuren, wie Salz- oder Essigsäure, Niederalkanole, z.B. Ethanol, und/oder Amine bzw. Ammoniak in Frage kommen. Solche Systeme sind beispielsweise Natrium/Ammoniak, Zink/Salz- oder Essigsäure oder Zink/Ethanol.

In bevorzugten Ausführungsformen dieses Verfahrens werden beispielsweise Hydroxy, mit einer Niederalkancarbonsäure verestertes Hydroxy, substituiertes Thio bzw. Seleno und disubstituiertes Amino durch Hydrierung mit Wasserstoff und z.B. folgende Reste durch Hydridübertragende Reagenzien reduziert: Halogen (z.B. mit Tributylzinnhydrid, Lithiumaluminiumhydrid, Natriumborhydrid oder Natriumcyanoborhydrid), Sulfonyloxy (z.B. mit Natriumcyanoborhydrid, Lithiumaluminiumhydrid oder Lithiumtriethylborhydrid), Niederalkanoyloxy (z.B. mit Lithiumaluminiumhydrid), Di-sulfonyloxy-amino (z.B. Natriumborhydrid).

Bevorzugt werden benzylische durch Wasserstoff ersetzbare Reste ($X_3$) reduktiv durch Wasserstoff ersetzt.

Ausgehend von Verbindungen der Formel III, worin die Variablen $X_2$ und $X_3$ insbesondere substituierte Thiogruppen, z.B. solche der vorstehend genannten Art, insbesondere Niederalkylthio, darstellen, können diese beispielsweise jeweils durch Hydrierung mit Wasserstoff in Gegenwart eines der genannten Hydrierungskatalysatoren, insbesondere von Raney-Nickel, durch Wasserstoff ersetzt werden, wobei in situ eine Verbindung der Formel III gebildet werden kann, in der $X_2$ und $X_4$ Wasserstoff sind und $X_3$ entsprechend substituiertes Thio bedeutet.

In Verbindungen der Formel III, worin $X_2$ und $X_3$ gemeinsam eine zweiwertige, durch zwei Wasserstoffatome ersetzbare Gruppe bedeuten, und $X_4$ Wasserstoff ist, stellt diese zweiwertige Gruppe beispielsweise die Oxo- oder eine gegebenenfalls substituierte Hydrazonogruppe dar.

Die Oxogruppe kann beispielsweise analog der Clemmensen-Reduktion mit amalgamiertem Zink in Gegenwart einer Protonsäure, wie Salzsäure, direkt durch zwei Wasserstoffatome ersetzt werden. Die Oxogruppe kann ferner mit Hydrazin in Gegenwart eines Alkalimetallhydroxids, gemäss Wolff-Kishner, oder in einem hochsiedenden Lösungsmittel, insbesondere in einem entsprechenden Ether, bei erhöhten Temperaturen, nach Huang-Minlon, oder in Gegenwart eines Alkalimetallalkanolats, insbesondere Kalium-tert.-butylat in Dimethylsulfoxid, nach Gram, durchgeführt werden. Dabei kann intermediär das Hydrazon ($X_2$, $X_3$ gemeinsam: $=N-NH_2$) gebildet werden, welches zweckmässig nicht isoliert und aus welchem unter den Reaktionsbedingungen Stickstoff abgespalten wird.

Bedeuten $X_2$ und $X_3$ gemeinsam beispielsweise substituiertes Hydrazono, wie Sulfonylhydrazono, z.B. p-Toluol-sulfonylhydrazono, so kann dieses beispielsweise mit einem Hydrid-übertragenden Reagenz, wie einem der vorstehend genannten, insbesondere Natriumborhydrid, Lithiumaluminiumhydrid oder Natriumcyanoborhydrid, durch zwei Wasserstoffatome ersetzt werden.

Im Verlaufe des Austauschs der durch Wasserstoff ersetzbaren Reste bzw. Gruppen können als Nebenreaktionen Eliminierungen von $H-X_3$ bzw. $H-X_4$ auftreten. Diese können nach Massgabe der pH-Verhältnisse zurückgedrängt werden. So kann insbesondere in neutralem Medium, welches z.B. durch geeignete Puffersysteme erhalten wird, gearbeitet werden. Ebenso kann bei Wahl von Ausgangsmaterial der Formel III, worin $X_3$ bzw. $X_4$ einwertige, z.B. durch Basen eliminierbare Gruppen bedeuten, wie verestertes Hydroxy, vorzugsweise in saurem Milieu gearbeitet und die Reduktion von solchen Verbindungen der Formel

III, worin die entsprechenden durch Wasserstoff ersetzbaren Reste in einer Nebenreaktion unter sauren Bedingungen eliminiert werden können, in erster Linie Hydroxy, bevorzugt im alkalischen Milieu durchgeführt werden.

Ein weiterer durch Wasserstoff ersetzbarer Rest $X_3$ bzw. $X_4$ ist die Carboxygruppe. Die Decarboxylierung von entsprechenden Verbindungen der Formel III kann üblicherweise bei erhöhten Temperaturen, beispielsweise ab einer Temperatur von etwa 50°C, insbesondere in einem Temperaturbereich von 100° bis etwa 300°C, durchgeführt werden. Die Decarboxylierung kann beispielsweise durch die Gegenwart von Basen, beispielsweise von hochsiedenden Stickstoffbasen, z.B. Collidin, und/oder in Gegenwart von Edelmetallen, wie Kupfer, unterstützt werden.

Die Reduktion der in Verbindungen der Formel III durch $X_3$ und $X_4$ dargestellten zusätzlichen Bindung erfolgt in erster Linie durch Hydrierung in Gegenwart eines der genannten Hydrierungskatalysatoren, ferner mit Hilfe eines Hydrid-übertragenden Reagenz, z.B. eines Alkalimetallborhydrids, wie Kaliumborhydrid.

Im Verlauf der vorstehend genannten Reduktionsreaktionen, insbesondere bei Hydrierungen, können gegebenenfalls vorhandene Mehrfachbindungen, z.B. solche bei entsprechenden Resten $R_1$, $R_2$ sowie Substituenten des Rings A, ebenfalls reduziert werden.

Das Ausgangsmaterial der Formel III, worin $X_2$ $R_1$ ist, $X_3$ Hydroxy bedeutet bzw. worin $X_2$ und $X_3$ gemeinsam die Oxogruppe darstellen und $X_4$ jeweils Wasserstoff ist, kann z.B. hergestellt werden, indem man ein 3-Nitrophenol in Gegenwart einer Lewissäure mit Acetylchlorid acetyliert, anschliessend die Nitrogruppe durch Hydrierung in Gegenwart eines Hydrierungskatalysators zur Aminogruppe reduziert und diese gemäss Variante (a) in $R_2$ überführt. Die so erhältlichen Verbindungen der Formel

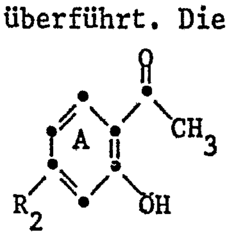

(IIIa)

werden z.B. bromiert und in Gegenwart einer Base, wie Natriumacetat, zu Verbindungen der Formel III cyclisiert, worin $X_2$ und $X_3$ die Oxogruppe bilden und $X_4$ Wasserstoff ist. Diese Zwischenverbindungen können einerseits mit Verbindungen der Formel $R_1$-Mg-Hal, wobei $R_1$ von Wasserstoff verschieden ist, analog der Grignard-Reaktion zu Verbindungen der Formel III umgesetzt werden, worin $X_2$ von Wasserstoff verschiedenes $R_1$ ist, $X_3$ Hydroxy bedeutet und $X_4$ Wasserstoff ist, oder andererseits mit einem Hydrid-übertragenden Reagenz, z.B. Natriumborhydrid, zu Verbindungen der Formel III reduziert werden, worin $X_2$ Wasserstoff bedeutet, $X_3$ Hydroxy ist und $X_4$ Wasserstoff darstellt.

Weitere Verbindungen der Formel III, worin einer der Reste $X_3$ und $X_4$ für einen durch Wasserstoff ersetzbaren Rest steht, können beispielsweise ausgehend von Verbindungen der Formel III erhalten werden, worin einer der Reste $X_3$ und $X_4$ Hydroxy bedeutet. Die Hydroxygruppe kann beispielsweise durch übliche anorganische Säurehalogenide, wie den Phosphorhalogeniden, Phosphortrichlorid, -bromid, Phosphoroxychlorid oder Thionylchlorid, durch Halogen substituiert, durch Behandlung mit einer Sulfonsäure oder organischen Carbonsäure oder einem reaktionsfähigen Derivat davon zu Sulfonyloxy bzw. Acyloxy verestert oder durch Reaktion mit einem Mercaptan in Gegenwart einer Lewissäure zu einem substituierten Thiorest umgesetzt werden. Halogen kann wiederum z.B. durch Behandeln mit Kupfer(I)cyanid in Cyano übergeführt werden, aus welchem durch Hydrolyse die Carboxygruppe $X_3$, ferner $X_4$ erhalten werden kann. Carboxyverbindungen ihrerseits können auf dem Wege der Umlagerung, z.B. nach Curtius, Lossen, Schmidt oder Hofmann, in Amino übergeführt und anschliessend, z.B. durch übliche Alkylierung, disubstituiert werden.

Die durch Wasserstoff ersetzbaren Reste $X_3$ bzw. $X_4$ können auch in einem früheren Stadium der Reaktionssequenz eingeführt werden.

Variante (c):

Eine reaktionsfähige veresterte Hydroxygruppe $X_5$ bzw. $X_5'$ stellt z.B. eine insbesondere mit einer Mineralsäure, in erster Linie Halogenwasserstoffsäure, veresterte Hydroxygruppe dar und steht in erster Linie für Halogen, wie Chlor, Brom oder Iod, ferner für mit einer Sulfonsäure oder organischen Carbonsäure verestertes Hydroxy, z.B. (Trifluor-)Methan- oder p-Toluol-sulfonyloxy.

Veräthertes Hydroxy bedeutet in erster Linie Niederalkoxy.

Ein metallischer Rest $X_5$ bzw. $X_5'$ steht z.B. für ein ein- oder zweiwertiges Metall, wie ein Alkalimetall, insbesondere Lithium, oder für einen zweiwertigen Metallrest, wie einen Cadmiumrest oder insbesondere Magnesium-, z.B. Magnesiumhalogenidrest.

Bevorzugt werden solche Verbindungen der Formeln IVa bzw. IVb eingesetzt, worin $X_5$ bzw. $X_5'$ Halogen bedeutet.

Bei Verwendung von Alkalimetall-, insbesondere Lithiumverbindungen der Formeln IVa bzw. IVb wird vorteilhaft bei erniedrigten Temperaturen, z.B. zwischen 10°C und -78°C, gearbeitet.

Die Umsetzung von Benzofuranen der Formel IVa mit Verbindungen der Formel IVb, worin $X_5$ für gegebenenfalls veräthertes Hydroxy steht und $X_5'$ ein Alkalimetall, insbesondere Lithium, bedeutet, wird zweckmässig unter Zusatz von Nickel- oder Palladiumverbindungen, z.B. Nickel(II)-chlorid, durchgeführt.

Die Reaktion von Verbindungen der Formeln IVa und IVb, worin $X_5$ und $X_5'$ jeweils Halogen bedeuten, kann beispielsweise in Anlehnung an die Wurtz- bzw. Fittig-Reaktion, beispielsweise in Gegenwart eines Alkalimetalls, wie Natrium, erfolgen.

Die Herstellungsweise von Ausgangsmaterial der Formel IVa wird beispielsweise im Zusammenhang mit der Herstellung von Ausgangsverbindungen der Formel III bzw. VI [Varianten b) bzw. e)] beschrieben bzw. erfolgt nach an sich bekannten Methoden.

Variante (d):

Im Ausgangsmaterial der Formel V, worin $X_6$ für die Gruppe der Formel $-CH(R_1)-CH_2-X_6'$ steht und $X_7$ Hydroxy ist, bedeutet die Abgangsgruppe $X_6'$ beispielsweise gegebenenfalls reaktionsfähiges, z.B. mit einer Mineralsäure, Sulfonsäure oder organischen Carbonsäure, verestertes Hydroxy, wie Halogen, Sulfato, gegebenenfalls substituiertes Phenyl- bzw. Niederalkansulfonyloxy oder gegebenenfalls substituiertes Niederalkanoyloxy. Insbesondere sind Chlor, Brom, Sulfato, p-Toluolsulfonyloxy oder Acetyloxy zu nennen.

Die Cyclisierung wird beispielsweise in Gegenwart eines Kondensationsmittels, wie einer der vorstehend genannten starken Protonsäuren oder insbesondere einer der vorstehend aufgeführten Basen, durchgeführt.

So können beispielsweise Ausgangsverbindungen der Formel V, worin $X_6$ für die Gruppe der Formel $-CH(R_1)-CH_2-X_6''$ und $X_6''$ für Halogen, wie Brom, steht, in Gegenwart eines basischen Kondensationsmittels, z.B. DBU, cyclisiert werden.

Bei der Cyclisierung von Ausgangsverbindungen der Formel V, worin $X_6$ für die Diazoniumgruppe steht und $X_7$ eine Gruppe der Formel $-O-CH_2-CH=R_1'$ bedeutet, wird beispielsweise in Gegenwart von reduzierenden Systemen, wie von Titan(III)halogeniden, z.B. Titan(III)chlorid, oder von Systemen aus Metall, wie Alkalimetall, und Niederalkanol gearbeitet. Sofern $X_6$ Halogen, insbesondere Iod, bedeutet, wird die Cyclisierung vorzugsweise in Gegenwart eines Hydrid-übertragenen Reagenz, insbesondere Tributylzinnhydrid, durchgeführt.

Die Cyclisierung von Verbindungen der Formel V, worin $X_6$ Wasserstoff ist und $X_7$ für die Gruppe der Formel $-O-CH_2-CO-R_1$ steht, kann beispielsweise in Gegenwart eines Kondensationsmittels, wie einer der bereits genannten starken Protonsäure, z.B. p-Toluolsulfonsäure, Salzsäure oder Schwefelsäure, oder eines Titan(III)halogenids, z.B. Titan(III)-chlorid, durchgeführt werden. Dabei können auch solche Verbindungen der Formel V eingesetzt werden, in denen die Carbonylgruppe des Restes $X_7$ in derivatisierter, z.B. acetalisierter, Form vorliegt. Entsprechende Acetale werden beispielsweise aus Niederalkanolen oder Niederalkandiolen bzw. entsprechenden Mercaptanen gebildet.

Die Gruppe der Formel $-O-CH_2-CO-R_1$ $X_7$ kann auch in der entsprechenden tautomeren Enolform ($X_7$ = $-O-CH=C(OH)-R_1$) vorliegen.

In Verbindungen der Formel V, worin $X_6$ für die Gruppe der Formel $-C(R_1)=CH-X_6''$ steht und $X_7$ Hydroxy bedeutet, stellt die Abgangsgruppe $X_6''$ in erster Linie Niederalkoxy dar, hat ferner die für $X_6'$ angegebenen Bedeutungen.

Die entsprechende Cyclisierung kann ebenso wie die unmittelbar vorstehend beschriebene Cyclisierung in Gegenwart eines, insbesondere sauren, Kondensationsmittels, vorteilhaft mit Hilfe von Perchlorsäure, durchgeführt werden.

Zur Herstellung von Ausgangsmaterial der Formel V, worin $X_6$ für die Gruppe der Formel $-CH(R_1)-CH_2-X_6'$ steht und $X_7$ Hydroxy ist, geht man beispielsweise von einem 1-Niederalkoxy-3-nitro-benzol aus und acyliert dieses in Gegenwart einer Lewissäure z.B. mit einem Acetylhalogenid. Dabei kann die Ethergruppierung gespalten werden; sie wird zweckmässigerweise jedoch wieder gebildet. In dem so erhältlichen 1-Acetyl-2-niederalkoxy-4-nitro-benzol kann die Nitrogruppe beispielsweise durch Hydrierung in Gegenwart eines Hydrierungskatalysators in die Aminogruppe und diese analog Variante (a) in den

Rest $R_2$ übergeführt werden. Eine so erhältliche Verbindung der Formel

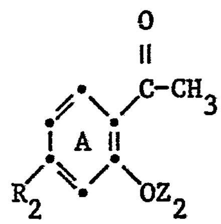

(Va)

kann im folgenden Reaktionsschritt in Anlehnung an die Willgerodt- bzw. Kindler-Reaktion z.B. mit Schwefel und Morpholin zum entsprechenden Thiomorpholid umgesetzt werden, welches anschliessend hydrolysiert wird. Gewünschtenfalls kann durch Umsetzung mit einem Halogenid der Formel $R_1$-Hal in Gegenwart einer starken Base, wie Natriumamid, der von Wasserstoff verschiedene Rest $R_1$ eingeführt werden. Anschliessend wird der Ether gespalten, z.B. durch Behandeln mit einer starken Halogenwasserstoffsäure oder einer Lewissäure, wobei gleichzeitig Cyclisierung in Verbindungen der Formel

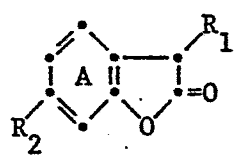

(Vb)

erfolgen kann. Der Lactonring wird anschliessend geöffnet, z.B. durch Behandeln mit einer Base. In resultierenden Verbindungen der Formel

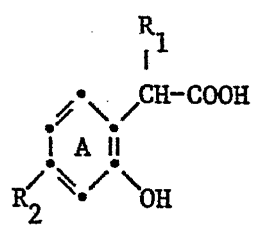

(Vc)

kann die Carboxygruppe reduktiv, z.B. mit Lithiumaluminiumhydrid, in die Hydroxymethylgruppe übergeführt werden. Gewünschtenfalls überführt man in z.B. erhältlichen Ausgangsverbindungen der Formel V, worin $X_6$ für die Gruppe der Formel $-CH(R_1)-CH_2-X_6'$ steht

und $X_6'$ Hydroxy ist, dieses in andere Abgangsgruppen $X_6'$, beispielsweise in Halogen,z.B. durch Umsetzung mit einem geeigneten Halogenierungsreagenz, wie mit einem Bortrihalogenid.

Zum Ausgangsmaterial der Formel V, worin $X_6$ die Diazoniumgruppe bzw. Halogen bedeutet und $X_7$ für die Gruppe der Formel $-O-CH_2-CH=R_1'$ steht, kann man beispielsweise gelangen, indem man in ein 3-Aminophenol analog Variante (a) den Rest $R_2$ einführt und die resultierende Verbindung der Formel

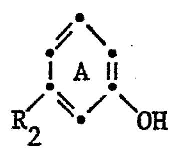

(Vd)

nitriert, die Nitrogruppe durch Reduktion, z.B. mit Eisen in Salzsäure oder Hydrierung in Gegenwart eines Hydrierungskatalysators, in die Aminogruppe überführt. Die Umsetzung mit einer Verbindung der Formel $Hal-CH=CH-R_1$ in Gegenwart einer Base kann beispielsweise zu Verbindungen der Formel

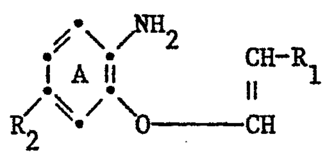

(Ve)

oder Isomeren derselben führen. Die Aminogruppe wird anschliessend, z.B. mit salpetriger Säure, diazotiert und die Diazoniumgruppe gewünschtenfalls z.B. analog der Sandmeyer-Reaktion mit einem Kupfer-(I)halogenid behandelt und somit durch ein Halogenatom ausgetauscht.

Zur Herstellung von Ausgangsverbindungen der Formel V, worin $X_6$ Wasserstoff ist und $X_7$ für die Gruppe der Formel $-O-CH_2-CO-R_1$ steht, geht man beispielsweise von einer Verbindung der Formel Vd aus und setzt diese z.B. mit einer Verbindung der Formel $Hal-CH_2-CO-R_1$ um.

Ausgangsmaterial der Formel V, worin $X_6$ die Gruppe der Formel $-C(R_1)=CH-X_6''$ darstellt und $X_6''$ und $X_7$ Hydroxy sind, kann beispielsweise erhalten werden, indem man z.B. eine Verbindung der Formel

Vc in das Carbonsäurehalogenid überführt, z.B. mit Oxalylchlorid, und die Halogencarbonylgruppe z.B. nach Rosenmund, durch Hydrierung in Gegenwart von Palladium auf Bariumsulfat, oder mit Lithium-tri-tert.-butoxy-aluminiumhydrid, bei tiefen Temperaturen in die Formylgruppe, die im Gleichgewicht mit der tautomeren Enolgruppe steht, überführt.

Variante (e):

In Verbindungen der Formel (VI) können die durch Wasserstoff ersetzbaren Reste $X_8$ bzw. $X_9$ beispielsweise für die in Variante (b) für $X_3$ bzw. $X_4$ angegebenen Bedeutungen haben. Sie stellen jedoch insbesondere Hydroxy, verestertes Hydroxy bzw. substituiertes Thio sowie in erster Linie Carboxy dar. Entsprechend können derartige Reste, z.B. wie in Variante (b) angegeben, durch Wasserstoff ersetzt werden. Bevorzugt sind entsprechende, von Carboxy verschiedene Reste in Position 3 des Ringsystems und Carboxy insbesondere in Position 2 lokalisiert.

Verbindungen der Formel VI, worin $X_8$ Carboxy bedeutet und $X_9$ Wasserstoff ist, sind beispielsweise zugänglich, indem man z.B. eine Verbindung der Formel Vd in Gegenwart einer Base zunächst mit einer Verbindung der Formel Hal-$CH_2$-$COOZ_2$ und anschliessend mit einem Oxalsäurediester umsetzt. Nach Hydrolyse der Esterfunktionen und Cyclisierung mit Hilfe einer starken Protonsäure können so erhältliche Verbindungen der Formel

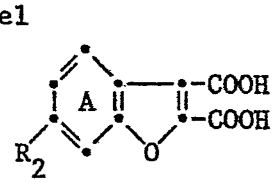

(VIa)

in üblicher Weise zu den entsprechenden Verbindungen der Formel VI, worin $X_8$ Carboxy bedeutet und $X_9$ Wasserstoff ist, decarboxyliert werden.

Zu Ausgangsverbindungen der Formel VI, worin $X_8$ Wasserstoff ist und $X_9$ Carboxy bedeutet, kann man gelangen, indem man beispielsweise eine Verbindung der Formel

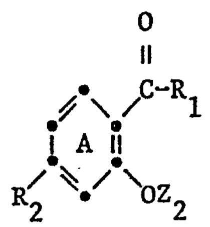

(VIb),

worin $Z_2$ Wasserstoff ist, in Gegenwart einer Base mit einem Ester der Formel Hal-$CH(COOH)_2$ und unter Erwärmen umsetzt und die resultierende Verbindung der Formel

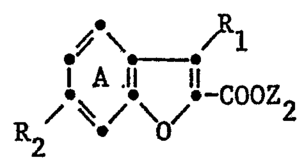

(VIc),

worin $Z_2$ z.B. Niederalkyl bedeutet, unter Bildung der freien Säure hydrolysiert.

Variante (f):

Abspaltbare Reste $X_{10}$ bzw. $X_{11}$ in Verbindungen der Formel VII sind beispielsweise gegebenenfalls, z.B. durch eine Mineralsäure, wie Halogenwasserstoff- oder Schwefelsäure, Sulfonsäuren, wie gegebenenfalls substituierte Phenyl- bzw. Niederalkansulfonsäuren, oder organische Carbonsäuren, wie gegebenenfalls substituierten Niederalkancarbonsäuren, verestertes Hydroxy, verethertes Hydroxy, wie Niederalkoxy, disubstituiertes Sulfonium, wie Diniederalkylsulfonium, tri-substituiertes Ammonium, wie Triniederalkylammonium, substituiertes Sulfinyl, wie gegebenenfalls substituiertes Phenyl- bzw. Niederalkansulfinyl, disubstituiertes N-Oxido-amino, wie Diniederalkyl-N-oxido-amino, oder Niederalkylthio-thiocarbonyloxy.

Die Eliminierung von $X_{10}$ - $X_{11}$ aus entsprechenden Verbindungen der Formel VII kann beispielsweise in Gegenwart einer Protonsäure, Lewissäure oder saurer anorganischer Salze ($X_{10}$ bzw. $X_{11}$ = Hydroxy), in Gegenwart einer Base ($X_{10}$ bzw. $X_{11}$ = verestertes oder verethertes Hydroxy, disubstituiertes Sulfonium oder trisubstituiertes

Ammonium) oder thermisch ($X_{10}$ bzw. $X_{11}$ = substituiertes Sulfinyl, disubstituiertes N-Oxidoamino oder Niederalkylthio-thiocarbonyloxy) erfolgen. Als Säuren bzw. Basen kommen insbesondere solche der vorstehend genannten Art, ferner als Basen Lithiumdiniederalkylamide, wie Lithiumdiethyl- oder Lithiumdipropylamid, in Frage. Die thermische Eliminierung erfolgt z.B. in einem Temperaturbereich von etwa 50° bis etwa 300°C.

Bedeuten $X_{10}$ und $X_{11}$ der Formel VII jeweils Wasserstoff, kann ein Molekül Wasserstoff unter Bildung einer zusätzlichen Bindung z.B. in Gegenwart eines Dehydrierungsmittels abgespalten werden. Solche Dehydrierungsmittel sind beispielsweise Hydrierungskatalysatoren, z.B. solche der vorstehend genannten Art, die gegebenenfalls auf einem geeigneten Trägermaterial aufgezogen sein können, z.B. Chinone, wie p-Benzochinone, z.B. Tetrachlor-p-benzochinon oder 2,3-Dichlor-5,6-dicyano-p-benzochinon, Anthrachinone oder Phenanthren-9,10-non. Ebenso können Selen bzw. Selenderivate, wie Selendioxid oder Diphenylselenium-bis-trifluoracetat, verwendet werden.

Bedeuten $X_{10}$ und $X_{11}$ der Formel VII jeweils Halogen wie Chlor, Brom oder Jod, kann ein Molekül Halogen unter Bildung einer zusätzlichen Doppelbindung z.B. unter reduktiven Bedingungen, wie in Gegenwart von unedlen Metallen, z.B. Zink oder Magnesium, von metallorganischen Reduktionssystemen, wie Alkalimetallsalz von Dimethylsulfoxid, oder Natriumiodid z.B. in Aceton, eliminiert werden.

Bedeuten $X_{10}$ und $X_{11}$ der Formel VII jeweils Carboxy, die Doppelbindung z.B. durch Oxidation mit einem geeigneten Oxidationsmittel, wie Blei (V)-acetat, z.B. in Gegenwart einer Base, wie Piperidin, eingeführt werden.

Die Ausgangsverbindungen der Formel VII bzw. ihre Vorstufen können z.B. analog der Herstellung von Verbindungen der Formel III (Variante b) hergestellt werden.

Variante (g):

Zwischenverbindungen der Formel VIII werden in üblicher Weise in situ gebildet und können z.B. unter den Reaktionsbedingungen für ihre Herstellung zu entsprechenden Verbindungen der Formel I isomerisiert werden. Werden die Verbindungen der Formel VIII isoliert, so kann man diese beispielsweise in Gegenwart einer Base, einer Säure oder thermisch isomerisieren. Diese Isomerisierung kann ebenfalls in Gegenwart von geeigneten Rhodiumverbindungen, wie Rhodium(III)halogeniden, z.B. Rhodiumtrichlorid, durchgeführt werden.

Die Bildung von Zwischenverbindungen der Formel VIII kann beispielsweise ausgehend von Verbindungen der Formel III erfolgen, worin $X_2$ und $X_3$ gemeinsam für die Oxogruppe stehen und $X_4$ Wasserstoff ist, indem man diese z.B. in Analogie zur Wittig-Reaktion bzw. Horner-Variante mit Verbindungen der Formeln $(C_6H_5)_3P=R_1'$ bzw. $(Z_2O)_2P(=O)-R_1$ umsetzt, wobei $R_1'$ ein dem Rest $R_1$ entsprechender doppelt gebundener aliphatischer Rest darstellt und $Z_2$ z.B. Niederalkyl bedeutet.

Die Zwischenprodukte der Formel VIII können ebenfalls gebildet werden, indem man beispielsweise eine Verbindung der Formel III, worin $X_2$ und $X_4$ Wasserstoff bedeuten und $X_3$ Halogen, insbesondere Brom, ist, zunächst einerseits mit Triphenylphosphin bzw. andererseits mit einem Phosphorigsäureester der Formel $(Z_2O)_3P=O$ umsetzt und anschliessend jeweils mit einer starken Base, wie n-Butyl-lithium, Natriumamid oder Natrium-methylsulfinylmethanid, in das jeweilige Alkylidenphosphoran bzw. den Phosphorigsäureester überführt. Diese reaktiven Phosphor-organischen Verbindungen werden im nächsten Reaktionsschritt mit einem Aldehyd der Formel $R_1''$-CHO, worin $R_1''$ ein dem Rest $R_1$ entsprechender, um ein C-Atom verminderter aliphatischer Rest bzw. Wasserstoff bedeutet, umgesetzt.

Die Erfindung betrifft insbesondere die in den Beispielen illustrierten Verfahren zur Herstellung der erfindungsgemässen Verbindungen sowie Formulierungsverfahren.

Eine erfindungsgemäss erhältliche Verbindung kann in an sich bekannter Weise in eine andere erfindungsgemässe Verbindung umgewandelt werden.

Weist der aromatische Ring A als Substituenten ein Wasserstoffatom auf, so kann dieses mit Hilfe eines Halogenierungsmittels in üblicher Weise durch ein Halogenatom ersetzt werden, z.B. durch Bromierung mit Brom, Hypobromsäure, Acylhypobromiten oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethyl-hydantoin, 2,4,4,6-Tetrabrom-2,5-cyclohexandien-1-on, bzw. durch Chlorierung, vorteilhaft mit elementarem Chlor, z.B. in einem halogenierten Kohlenwasserstoff, wie Chloroform, und unter Kühlen, z.B. auf etwa -10° bis etwa +10°C.

Ferner kann der Benzoteil des Ringsystems beispielsweise mit einem Niederalkanol bzw. einem Niederalkylhalogenid oder einem Phosphorsäureniederalkylester in Gegenwart von Lewis-Säuren alkyliert werden (Friedel-Crafts-Alkylierung). In einer Verbindung der Formel (I), worin der aromatische Ring A Brom enthält, kann beispielsweise das Brom durch Umsetzung mit einem Niederalkylbromid in Gegenwart eines Alkalimetalls durch Niederalkyl ersetzt werden.

Enthält der aromatische Ring A als Substituenten ein Wasserstoffatom, so kann dieser in an sich bekannter Weise durch eine Acylgruppe ausgetauscht werden. So kann beispielsweise die Einführung der Acylgruppe analog der Friedel-Crafts-Acylierung (cf. G.A. Olah, Friedel-Crafts and Related Reactions, Vol.I, Intersience, New York, 1963-1965) durchgeführt werden, z.B. durch Umsetzung eines reaktiven funktionellen Acylderivates, wie eines Halogenids oder Anhydrids, einer organischen Carbonsäure in Gegenwart einer Lewis-Säure, wie Aluminium-, Antimon-(III)- oder (V)-, Eisen(III)-, Zinn(II)-, Zink(II)-chlorid oder Bortrifluorid.

Enthält der aromatische Ring A als Substituenten Hydroxy, so lässt sich dieses nach an sich bekannter Weise verethern. Die Umsetzung mit einer Alkoholkomponente, z.B. mit einem Niederalkanol, wie Ethanol,

in Gegenwart von Säuren, z.B. Mineralsäure, wie Schwefelsäure, oder von Dehydratisierungsmitteln, wie Dicyclohexylcarbodiimid, führt z.B. zu Niederalkoxy. Umgekehrt kann man Ether in Phenole spalten, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Pyridin-hydrochlorid oder Thiophenol durchführt.

Weiter lässt sich Hydroxy in Niederalkanoyloxy umwandeln, beispielsweise durch Umsetzung mit einer gewünschten Niederalkancarbonsäure, wie Essigsäure, oder einem reaktionsfähigen Derivat davon, beispielsweise in Gegenwart einer Säure, wie einer Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Falls der Ring A durch Niederalkylthio substituiert ist, kann man dieses auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig-, m-Chlorperbenzoe- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der VII. Nebengruppe, z.B. Vanadium-, Molybdän- oder Wolframoxid, zu nennen

sind. Die Oxidation wird unter milden Bedingungen, z.B. bei Termperaturen von -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hiebei üblicherweise das Oxidationsmittel im Ueberschuss ein.

Wasserstoff $R_1$ kann beispielsweise durch Alkylierung, z.B. mit einem reaktionsfähigen veresterten Alkohol der Formel $R_1$-OH, z.B. einem entsprechenden Halogenid oder einem Sulfonyloxy-Derivat, insbesondere in Gegenwart einer starken Base, in Alkyl $R_1$ übergeführt werden. Die Alkylierung kann auch z.B. durch eine vorgelagerte Substitution von Wasserstoff $R_1$ durch Halogen und anschliessende Umsetzung mit Metallverbindungen der Formel $R_1$-Me erfolgen, wobe Me z.B. für ein Alkalimetall, Erdalkalimetallhalogenid oder Li-Cu steht.

Enthalten die Verbindungen der Formel (I) ungesättigte Reste, wie Niederalkenyl oder Niederalkenylengruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet sind, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80° bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Umgekehrt können in cyclischen Systemen $R_2$ eine oder mehrere Mehrfachbindungen eingeführt werden. Hierzu verwendet man geeignete Dehydrierungsmittel, beispielsweise Nebengruppenelemente, vorzugsweise solche

der VIII. Nebengruppe des Periodensystems, z.B. Palladium oder Platin, oder entsprechende Edelmetallderivate, z.B. Ruthenium-triphenylphosphidchlorid, wobei die Mittel auf geeigneten Trägermaterialien aufgezogen sein können. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone oder Phenanthren-9,10-chinon. Ferner können N-Halogensuccinimide, wie N-Chlorsuccinimid, Manganverbindungen, wie Bariummanganat oder Mangandioxid, und Selenderivate, wie Selendioxid oder Diphenylselenium-bis-trifluoracetat, verwendet werden.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften in freier Form oder in Form von Salzen erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Ueberführung in diastereomere Salze oder Ester, z.B. durch Umsetzung eines sauren Endstoffs mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Derivates bzw. Salzes, und/oder seiner Racemate bzw. Antipoden, verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei $R_1$, $R_2$ und A die für die jeweils bevorzugten Verbindungsgruppen der Formel I

angegebenen Bedeutungen haben.

Die Erfindung betrifft ebenfalls die Verwendung der Verbindungen der Formel (I) oder von pharmazeutisch verwendbaren Salzen von solchen Verbindungen mit salzbildenden Eigenschaften, insbesondere als pharmakologische, in erster Linie antiinflammatorisch, analgetisch und/oder antipyretisch wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere zur Behandlung von Erkrankungen des rheumatischen Formenkreises, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die die erfindungsgemässe Verbindung oder pharmazeutisch verwendbare Salze davon enthalten, handelt es sich um solche zur enteralen, wie oralen oder rektalen, und parenteralen Verabreichung, ferner zur topischen Anwendung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand, sowie von der Applikationsweise ab.

Die neuen pharmazeutischen Präparate enthalten z.B. von etwa 10% bis etwa 80%, vorzugsweise von etwa 20% bis etwa 60% des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw.

Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Als topisch anwendbare pharmazeutische Präparate kommen in erster Linie Creme, Salben, Pasten, Schäume, Tinkturen und Lösungen in Frage, die von etwa 0,1 bis 5% des Wirkstoffs enthalten und ebenfalls in an sich bekannter Weise hergestellt werden können.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Appkikation eine ungefähre Tagesdosis von etwa 100 bis etwa 600 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: Zu einer Lösung von 2,50 g (7,8 mMol) 1-Brom-2-[5-chlor-2-hydroxy-4-(piperidin-1-yl)-phenyl]-ethan in 50 ml abs. Methylenchlorid werden bei 0° 1,2 ml (8,0 mMol) 1,8-Diazabicyclo[5.4.0]undec-7-en (DBU) gegeben und anschliessend während 3 Stunden bei Raumtemperatur gerührt. Das Reaktionsgemisch wird mit gesättigter Natriumchlorid-Lösung gewaschen, die organischen Phasen vereinigt, über Natriumsulfat getrocknet und am Vakuumrotationsverdampfer eingeengt. Der Rückstand wird an Kieselgel mit dem Laufmittel Methylenchlorid chromatographiert. Umkristallisation aus Petrolether ergibt 5-Chlor-6-(piperidin-1-yl)-2,3-dihydrobenzofuran vom Smp. 38-40°.

Das Ausgangsmaterial kann folgendermassen hergestellt werden:
In einer auf 0° gekühlten Suspension von 173,3 g(1,3 Mol) Aluminiumtrichlorid in 600 ml abs. Methylenchlorid werden vorsichtig 177 g (1,0 Mol) 3,4-Dichloranisol [H. Jamarlik et al, Comptes Rendus Acad. Sci. Ser. C 273 (25), 1756 (1971)] gegeben. Anschliessend werden 85 ml (1,2 Mol) Acetylchlorid zugetropft und während 90 Minuten bei 0° rühren gelassen. Das Reaktionsgemisch wird auf Eis gegossen und mit Methylenchlorid extrahiert. Die organischen Extrakte werden mit Wasser gewaschen, vereinigt, über Natriumsulfat getrocknet und am Vakuumrotationsverdampfer eingedampft. Nach Umkristallisation aus Ether/Petrolether oder Methanol/Wasser erhält man das 4,5-Dichlor-2-methoxy-acetophenon vom Smp. 93-95°.

Eine Lösung von 76,7 g (0,35 Mol) 4,5-Dichlor-2-methoxy-acetophenon in 750 ml Piperidin wird während 7 Stunden bei 170° im Autoklaven gehalten. Das Reaktionsgemisch wird eingedampft, in Essigester aufgenommen und mit Wasser gewaschen. Die Essigester-Extrakte werden vereinigt, über Natriumsulfat getrocknet und am Vakuumrotationsverdampfer eingedampft. Der Rückstand wird an Kieselgel mit Methylenchlorid chromatographiert. Man erhält so das 5-Chlor-2-hydroxy-4-(piperidin-1-yl)-acetophenon vom Smp. 68-70°.

Eine Lösung von 32,5 g (128 mMol) 5-Chlor-2-hydroxy-4-(piperidin-1-yl)-acetophenon mit 75 ml (166 mMol) einer etwa 40%igen methanolischen

Lösung von Benzyltriethylammoniumhydroxid (Triton B) in 65 ml Tetrahydrofuran wird auf 0° abgekühlt. Während ca. 6 Minuten werden 14,6 ml (154 mMol) Dimethylsulfat so zugetropft, dass die Innentemperatur nicht über 5° steigt. Das Reaktionsgemisch wird noch 1 Stunde bei 0° gerührt, dann während etwa 30 Minuten unter Rückfluss gekocht. Das Reaktionsgemisch wird nun auf 400 ml Wasser gegossen und mit Essigester extrahiert. Die vereinigten Essigesterphasen werden mit Wasser gewaschen, über Natriumsulfat getrocknet und am Vakuumrotationsverdampfer eingedampft. Der Rückstand wird aus Methylenchlorid/Hexan umkristallisiert, und man erhält das 5-Chlor-2-methoxy-4-(piperidin-1-yl)-acetophenon vom Smp. 119-120°.

Eine Lösung von 18,2 g (68 mMol) 5-Chlor-2-methoxy-4-(piperidin-1-yl)-acetophenon und 4,36 g (136 mMol) Schwefel in 68 ml Morpholin wird während 5 Stunden bei 90° gehalten. Das Reaktionsgemisch wird abgekühlt, mit Essigester verdünnt und mit Wasser gewaschen. Die vereinigten Essigester-Extrakte werden über Natriumsulfat getrocknet und am Vakuumrotationsverdampfer eingedampft. Nach Umkristallisation aus Methylenchlorid/Methanol erhält man das 5-Chlor-2-methoxy-4-(piperidin-1-yl)-phenylthioessigsäuremorpholinamid vom Smp. 137-139°.

Eine Lösung von 11,07 g (30 mMol) 5-Chlor-2-methoxy-4-(piperidin-1-yl)-phenylthioessigsäuremorpholinamid in 120 ml Eisessig und 30 ml konz. Salzsäure wird während 22 Stunden unter Rückfluss gekocht. Das Reaktionsgemisch wird abgekühlt, mit Wasser verdünnt und mit Methylenchlorid extrahiert. Die vereinigten Methylenchloridphasen werden mit Wasser gewaschen, über Natriumsulfat getrocknet und am Vakuumrotationsverdampfer eingedampft. Nach Chromatographieren an Kieselgel mit Chloroform/Methanol (19:1) erhält man die 5-Chlor-2-methoxy-4-(piperidin-1-yl)-phenylessigsäure, die nach Umkristallisation mit Methylenchlorid/Hexan bei 120-122° schmilzt.

Zu einer Suspension von 1,6 g (42 mMol) Lithiumaluminiumhydrid in 90 ml abs. Tetrahydrofuran wird während ca. 15 Minuten eine Lösung von 14,2 g (50 mMol) 5-Chlor-2-methoxy-4-(piperidin-1-yl)-phenylessigsäure in 50 ml abs. Tetrahydrofuran getropft. Anschliessend wird noch 2 Stunden bei 50° nachgerührt. Nach Zugabe von 50 ml Wasser wird über Hyflo filtriert und mit THF nachgewaschen. Das Lösungsmittel wird am Vakuumrotationsverdampfer eingeengt und der Rückstand zwischen Ether und Wasser verteilt. Die Etherphasen werden vereinigt, über Natriumsulfat getrocknet und am Vakuumrotationsverdampfer eingeengt. Der Rückstand wird an Kieselgel mit dem Laufmittel Chloroform/Essigester (1:1) chromatographiert. Umkristallisation aus Ether/Petrolether ergibt 2-[5-Chlor 2-methoxy-4-(piperidin-1-yl)phenyl]-ethanol vom Smp. 56-57°.

Zu einer auf 0° gekühlten Lösung von 2,70 g (10 mMol) 2-[5-Chlor-2-methoxy-4-(piperidin-1-yl)-phenyl]-ethanol in 70 ml abs. Methylenchlorid werden während ca. 5 Minuten 5,0 ml Bortribromid zugetropft. Das Reaktionsgemisch wird anschliessend noch 5 Stunden bei Raumtemperatur gerührt, auf Eis gegossen, die entstandene Suspension mit festem Natriumcarbonat auf pH 6-7 gestellt und mit Methylenchlorid extrahiert. Die organischen Extrakte werden mit Wasser gewaschen, vereinigt, über Natriumsulfat getrocknet und am Vakuumrotationsverdampfer eingeengt. Umkristallisation aus Petrolether ergibt 1-Brom-2-[5-chlor-2-hydroxy-4-(piperidin-1-yl)-phenyl]-ethan, Smp. 115-116°.

Beispiel 2: Eine Lösung von 1,4 g (5,2 mMol) 5-Chlor-3-hydroxy-3-methyl-6-(piperidin-1-yl)-2,3-dihydro-benzofuran in 20 ml Essigsäure/5 N Salzsäure 1:1 wird während einer Stunde bei 85° gehalten. Das Reaktionsgemisch wird am Vakuumrotationsverdampfer eingeengt, der Rückstand mit Wasser versetzt, mit gesättigter Natriumcarbonat-Lösung auf pH 7 gestellt und mit Methylenchlorid extrahiert. Die organischen Phasen werden mit Wasser gewaschen, vereinigt, über Natriumsulfat getrocknet und am Vakuumrotationsverdampfer eingeengt. Nach Chromatographieren an Kieselgel mit dem Laufmittel Chloroform erhält man 5-Chlor-3-methyl-6-

(piperidin-1-yl)-benzofuran, das nach Umkristallisation aus Petrolether bei 82-84° schmilzt.

Das Ausgangsmaterial kann folgendermassen hergestellt werden:
Zu einer Lösung von 25,2 g (0,10 Mol) 5-Chlor-2-hydroxy-4-(piperidin-1-yl)-acetophenon in 240 ml Chloroform werden 6,0 ml (0,12 Mol) Brom während ca. 5 Minuten getropft. Das Reaktionsgemisch wird während 4 Stunden bei Raumtemperatur gerührt, mit Wasser versetzt und mit verdünnter Natriumbicarbonat-Lösung auf pH 6-7 gestellt. Die organische Phase wird abgetrennt, mit Wasser gewaschen, über Natriumsulfat getrocknet und am Vakuumrotationsverdampfer eingeengt. Der Rückstand wird an Kieselgel mit dem Laufmittel Toluol chromatographiert. Nach Umkristallisation aus Isopropanol/Petrolether erhält man das 5-Chlor-2-hydroxy-4-(piperidin-1-yl)-bromacetophenon vom Smp. 92-94°.

Zu einer auf -10° gekühlten Lösung von 10,0 g (30 mMol) 5-Chlor-2-hydroxy-4-(piperidin-1-yl)-bromacetophenon in 80 ml Dimethylformamid werden 10,0 g (120 mMol) Natriumacetat gegeben und während einer Stunde bei 0° gerührt. Das Reaktionsgemisch wird anschliessend mit Wasser verdünnt und dreimal mit Essigester extrahiert. Die organischen Phasen werden mit Wasser gewaschen, vereinigt, über Natriumsulfat getrocknet und am Vakuumrotationsverdampfer eingeengt. Man erhält so das 5-Chlor-6-(piperidin-1-yl)-benzofuran-3(2H)-on als Oel mit einem Rf von 0,3 (Kieselgel/Methylenchlorid), das ohne weitere Reinigung für die nächsten Umsetzungen eingesetzt wird.

Zu einer frisch hergestellten Grignard-Lösung aus 750 mg (30,8 mMol) Magnesium-Spänen und 4,0 g (28,2 mMol) Methyliodid in 30 ml abs. Ether wird bei Raumtemperatur eine Lösung von 5,03 g (20,0 mMol) rohem 5-Chlor-6-(piperidin-1-yl)-benzofuran-3(2H)-on in 30 ml abs. Ether getropft. Anschliessend wird während 45 Minuten unter Rückfluss gekocht und auf Eis/Ammoniumchlorid gegossen. Die Etherphase wird abgetrennt und die Wasserphase noch zweimal mit Ether extrahiert. Die organischen Phasen werden mit Wasser gewaschen, vereinigt, über Natriumsulfat getrocknet und am Vakuumrotationsverdampfer eingeengt. Nach Chromatographieren an Kieselgel mit Chloroform/Essigester (10:1) erhält man das 5-Chlor-3-hydroxy-3-methyl-6-(piperidin-1-yl)-2,3-dihydro-benzofuran als Oel mit einem Rf von 0,24 [Kieselgel:Chloroform/Essigester (10:1)].

Beispiel 3: Eine Lösung von 2,40 g (9,4 mMol) 5-Chlor-3-hydroxy-6-(piperidin-1-yl)-2,3-dihydro-benzofuran in 25 g Polyphosphorsäure wird während 20 Minuten bei 60° gehalten, dann mit Eis und Wasser versetzt und mit festem Natriumcarbonat auf pH 7 gestellt. Das Reaktionsgemisch wird zweimal mit Methylenchlorid extrahiert. Die organischen Phasen werden mit Wasser gewaschen, vereinigt, über Magnesiumsulfat getrocknet und am Vakuumrotationsverdampfer eingeengt. Der Rückstand wird an Kieselgel mit dem Laufmittel Methylenchlorid (Rf = 0,63) chromatographiert. Man erhält so das 5-Chlor-6-(piperidin-1-yl)-benzofuran als Oel, das im Kugelrohrofen bei 96-97°/1,5 Torr destilliert.

Das Ausgangsmaterial kann folgendermassen hergestellt werden: Zu einer auf 0° gekühlten Lösung von 3,3 g (13,1 mMol) 5-Chlor-6-(piperidin-1-yl)-benzofuran-3(2H)-on, gemäss Beispiel 2, in 80 ml Methanol werden 528 mg (13,1 mMol) Natriumborhydrid gegeben. Anschliessend wird noch 4 Stunden bei 0° weitergerührt. Das Reaktionsgemisch wird mit Wasser verdünnt, mit verdünnter Ammoniumchloridlösung auf pH 6-7 gestellt und mit Methylenchlorid mehrmals extrahiert. Die organischen Phasen werden mit Wasser gewaschen, vereinigt, über Natriumsulfat getrocknet und am Vakuumrotationsverdampfer eingeengt. Nach Chromatographieren an Kieselgel mit dem Laufmittel Methylenchlorid (Rf = 0,12) erhält man das 5-Chlor-3-hydroxy-6-(piperidin-1-yl)-2,3-dihydro-benzofuran als Oel.

Beispiel 4: Eine Lösung von 1,38 g (5,5 mMol) 5-Chlor-6-(piperidin-1-yl)-benzofuran-3(2H)-on in 20 ml Ethanol wird mit 200 mg Platinoxid versetzt und bei 3 Atmosphären hydriert. Der Katalysator wird abfiltriert und das Filtrat am Vakuumrotationsverdampfer eingeengt. Der Rückstand wird an Kieselgel mit dem Laufmittel Methylenchlorid chromatographiert. Umkristallisation aus Petrolether ergibt 5-Chlor-6-(piperidin-1-yl)-2,3-dihydrobenzofuran vom Smp. 38-40°.

Beispiel 5: Eine Lösung von 2,37 g (10,0 mMol) 5-Chlor-6-(piperidin-1-yl)-2,3-dihydro-benzofuran und 2,27 g (10,0 mMol) 2,3-Dichlor-5,6-dicyan-1,4-benzochinon in 200 ml Dioxan wird während 12 Stunden unter Rückfluss gekocht. Das Reaktionsgemisch wird abgekühlt, filtriert und das Filtrat am Vakuumrotationsverdampfer eingeengt. Der Rückstand wird an Kieselgel mit dem Laufmittel Methylenchlorid (Rf = 0,63) chromatographiert. Man erhält so das 5-Chlor-6-(piperidin-1-yl)-benzofuran als Oel, das im Kugelrohrofen bei 96-97°/1,5 Torr destilliert.

Beispiel 6: Eine Lösung von 1,42 g (5,60 mMol) 5-Chlor-3-hydroxy-6-(piperidin-1-yl)-2,3-dihydro-benzofuran in 20 ml Ethanol wird mit 200 mg Platinoxid versetzt und bei 3 Atmosphären hydriert. Der Katalysator wird abfiltriert und das Filtrat am Vakuumrotationsverdampfer eingeengt. Der Rückstand wird an Kieselgel mit dem Laufmittel Methylenchlorid chromatographiert. Umkristallisation aus Petrolether ergibt 5-Chlor—6-(piperidin-1-yl)-2,3-dihydro-benzofuran vom Smp. 38-40°.

Beispiel 7: Eine Lösung von 25,3 g (0,10 Mol) 5-Chlor-2-hydroxy-4-(piperidin-1-yl)-acetophenon, 18,4 g (0,11 Mol) Bromessigsäureethylester und 13,8 g (0,10 Mol) Kaliumcarbonat in 500 ml abs. Aceton werden während 12 Stunden unter Rückfluss gekocht. Das Reaktionsgemisch wird abgekühlt, filtriert und das Aceton am Vakuumrotationsverdampfer eingeengt. Der Rückstand wird in Wasser aufgenommen und mit Methylenchlorid extrahiert. Die organischen Phasen werden mit Wasser gewaschen, vereinigt, über Natriumsulfat getrocknet und am Vakuumrotationsverdampfer eingeengt. Kurzfiltration an Kieselgel mit dem Laufmittel Methylenchlorid/Petrolether (5:1) ergibt den 2-[2-Acetyl-4-chlor-5-(piperidin-1-yl)-phenoxy]-essigsäureethylester, der ohne weitere Reinigung mit 100 ml 2 N Natronlauge und 100 ml Ethanol versetzt und während 15 Minuten unter Rückfluss gekocht wird. Mit verdünnter Salzsäure wird auf pH 5-6 gestellt und mit Methylenchlorid extrahiert. Die organischen Phasen werden mit Wasser gewaschen, vereinigt, über Natriumsulfat getrocknet und am Vakuumrotationsverdampfer eingeengt. Der Rückstand wird in 100 ml Essigsäureanhydrid aufgenommen, mit 16 g (0,20 Mol) Natriumacetat versetzt und während 2 Stunden unter Rückfluss gekocht. Das Reaktionsgemisch wird abgekühlt, mit Wasser verdünnt, mit verdünnter Natriumcarbonat-Lösung auf pH 6-7 gestellt und mit Methylenchlorid extrahiert. Die organischen Phasen werden mit Wasser gewaschen, vereinigt, über Natriumsulfat getrocknet und am Vakuumrotationsverdampfer eingeengt. Nach Chromatographieren an Kieselgel mit dem Laufmittel Chloroform erhält man das 5-Chlor-3-methyl-6-(piperidin-1-yl)-benzofuran, das nach Umkristallisation aus Petrolether bei 82-84° schmilzt.

Beispiel 8: Eine Lösung von 2,51 g (10,0 mMol) 5-Chlor-6-(piperidin-1-yl)-benzofuran-3(2H)-on in 15 ml Essigsäureanhydrid und 15 ml Eisessig wird während 24 Stunden bei 100° gehalten. Anschliessend wird das Reaktionsgemisch auf Eis gegossen und mit Methylenchlorid extrahiert. Die organischen Phasen werden mit Wasser gewaschen, vereinigt, über Natriumsulfat getrocknet und am Vakuumrotationsverdampfer eingeengt. Das so erhaltene rohe 3-Acetoxy-5-chlor-6-(piperidin-1-yl)-benzofuran wird in 15 ml abs. Methanol gelöst, mit 250 mg Platinoxid versetzt und bei 3 Atmosphären und Raumtemperatur hydriert. Der Katalysator wird abfiltriert und das Filtrat am Vakuumrotationsverdampfer eingeengt. Der Rückstand wird an Kieselgel mit dem Laufmittel Methylenchlorid (Rf = 0,63) chromatographiert. Man erhält so das 5-Chlor-6-(piperidin-1-yl)-benzofuran als Oel, das im Kugelrohrofen bei 96-97°/1,5 Torr destilliert.

Beispiel 9: Eine Lösung von 250 mg (1,00 mMol) rohem 5-Chlor-2-hydroxy-6-(piperidin-1-yl)-2,3-dihydro-benzofuran in 2,5 g Polyphosphorsäure wird während 20 Minuten bei 60° gehalten, dann mit Eis und Wasser versetzt und mit festem Natriumcarbonat auf pH 7 gestellt. Das Reaktionsgemisch wird zweimal mit Methylenchlorid extrahiert. Die organischen Phasen werden mit Wasser gewaschen, vereinigt, über Natriumsulfat getrocknet und am Vakuumrotationsverdampfer eingeengt. Der Rückstand

wird an Kieselgel mit dem Laufmittel Methylenchlorid (Rf = 0,63) chromatographiert. Man erhält so das 5-Chlor-6-(piperidin-1-yl)-benzofuran als Oel, das im Kugelrohrofen bei 96-97°/1,5 Torr destilliert.

Das Ausgangsmaterial kann folgendermassen hergestellt werden:

Eine Lösung von 8,5 g (30 mMol) 5-Chlor-2-methoxy-4-(piperidin-1-yl)-phenylessigsäure in 150 ml 48%iger Bromwasserstoffsäure wird während 15 Stunden unter Rückfluss gekocht. Das Reaktionsgemisch wird abgekühlt, mit Wasser verdünnt und mit gesättigter Natriumbicarbonatlösung auf pH 3-4 gestellt. Anschliessend wird mit Essigester extrahiert, die vereinigten organischen Phasen mit Wasser gewaschen, über Natriumsulfat getrocknet und am Vakuumrotationsverdampfer eingedampft. Man erhält so einen dunkelgrauen Schaum von 5-Chlor-2-hydroxy-4-(piperidin-1-yl)-phenylessigsäure, der ohne Reinigung zum 5-Chlor-6-(piperidin-1-yl)-benzofuran-2(3H)-on umgesetzt wird.

Eine Lösung von 8,1 g (30 mMol) roher 5-Chlor-2-hydroxy-4-(piperidin-1-yl)-phenylessigsäure in 50 ml abs. Methylenchlorid wird bei Raumtemperatur während ca. 3 Minuten mit einer Lösung von 6,5 g (31,5 mMol) Dicyclohexylcarbodiimid in 40 ml abs. Methylenchlorid versetzt. Das Reaktionsgemisch wird während 30 Minuten bei Raumtemperatur gerührt. Der ausgefallene Dicyclohexylharnstoff wird abgenutscht und mit Methylenchlorid gewaschen. Das Filtrat wird am Vakuumrotationsverdampfer eingedampft und der Rückstand an Kieselgel mit dem Laufmittel Methylenchlorid/Hexan (1:1) chromatographiert. Umkristallisation aus Hexan ergibt 5-Chlor-6-(piperidin-1-yl)-benzofuran-2(3H)-on vom Smp. 129-131°.

Eine Lösung von 251 mg (1,00 mMol) 5-Chlor-6-(piperidin-1-yl)-benzofuran-2(3H)-on in 5 ml abs. Tetrahydrofuran wird unter Stickstoffatmosphäre auf -78° gekühlt und mit 0,84 ml (1,00 mMol) einer 20%igen Diisobutylaluminiumhydrid-Lösung in Toluol versetzt und während 30 Minuten bei -78° gerührt. Anschliessend werden 2 ml 2 N Schwefelsäure zugegeben und 15 Minuten bei -40° gerührt. Das Reaktionsgemisch wird

mit Wasser verdünnt und mit Methylenchlorid extrahiert. Die organischen Phasen werden mit Wasser gewaschen, vereinigt, über Natriumsulfat getrocknet und am Vakuumrotationsverdampfer eingeengt. Man erhält das 5-Chlor-2-hydroxy-6-(piperidin-1-yl)-2,3-dihydrobenzofuran als Oel, das ohne Reinigung für die nächsten Umsetzungen verwendet wird. Rf [Kieselgel, Chloroform/Essigester (3:1)] = 0,48.

<u>Beispiel 10</u>: Eine Lösung von 250 mg (1,00 mMol) rohem 5-Chlor-2-hydroxy-6-(piperidin-1-yl)-2,3-dihydro-benzofuran in 5 ml abs. Ethanol wird mit 5 mg Platinoxid versetzt und bei Raumtemperatur und 3 Atmosphären hydriert. Der Katalysator wird abfiltriert und das Filtrat am Vakuumrotationsverdampfer eingeengt. Der Rückstand wird an Kieselgel mit dem Laufmittel Methylenchlorid chromatographiert. Umkristallisation aus Petrolether ergibt 5-Chlor-6-(piperidin-1-yl)-2,3-dihydro-benzofuran vom Smp. 38-40°.

<u>Beispiel 11</u>: Ein Gemisch von 9,98 g (50 mMol) 1-(3-Amino-4-chlorphenoxy)-2-propanon, 22,9 g (0,10 Mol) 1,5-Dibrompentan, 12,9 g (0,10 Mol) N-Ethyldiisopropylamin und 60 ml Ethanol wird während 18 Stunden am Rückfluss gekocht. Der nach dem Eindampfen im Vakuum erhaltene Rückstand wird in Methylenchlorid aufgenommen und zweimal mit verdünnter Natriumbicarbonat-Lösung gewaschen. Die organische Phase wird getrocknet, eingedampft, das Rohprodukt in 250 ml Ethanol aufgenommen, mit 40 ml konz. Salzsäure versetzt und während 30 Stunden unter Rückfluss gekocht. Das Ethanol wird am Vakuumrotationsverdampfer eingeengt, der Rückstand mit Eiswasser verdünnt, mit 6 N Natronlauge alkalisch gestellt und mit Methylenchlorid extrahiert. Die organischen Phasen werden mit Wasser gewaschen, vereinigt, über Natriumsulfat getrocknet und am Vakuumrotationsverdampfer eingeengt. Nach Chromatographieren an Kieselgel mit dem Laufmittel Chloroform erhält man das 5-Chlor-3-methyl-6-(piperidin-1-yl)-benzofuran, das nach Umkristallisation aus Petrolether bei 82-84° schmilzt.

Beispiel 12: Ein Gemisch aus 9,1 g (50 mMol) 6-Amino-5-chlor-3-methylbenzofuran, 11,8 ml (0,1 Mol) 1,4-Dibrombutan, 17,1 ml (0,1 Mol) N-Ethyl-diisopropylamin und 60 ml Ethanol wird während 24 Stunden am Rückfluss gekocht und anschliessend im Vakuum eingedampft. Der Rückstand wird zwischen Methylenchlorid und gesättigter Natriumbicarbonatlösung verteilt. Das nach dem Trocknen und Eindampfen des Methylenchlorids erhaltene Rohprodukt wird mit Petrolether/Ether an Kieselgel chromatographiert. Anschliessende Destillation im Kugelrohr (150°/$10^{-1}$ Torr) ergibt 5-Chlor-3-methyl-6-(pyrrolidin-1-yl)-benzofuran vom Smp. 50-52°.

Das Ausgangsmaterial kann folgendermassen hergestellt werden:
Ein Gemisch aus 14,2 g (82 mMol) 4-Chlor-3-nitrophenol, 12,4 g (90 mMol) gemahlenem Kaliumcarbonat, 8,2 g (55 mMol) Natriumiodid, 8,3 g (90 mMol) Chloraceton und 220 ml Dimethylformamid wird während 3 Stunden bei Raumtemperatur gerührt. Anschliessend wird filtriert, der Rückstand mit Dimethylformamid nachgewaschen, die organische Phase im Vakuum eingedampft und das erhaltene Rohprodukt zwischen Wasser und Methylenchlorid verteilt. Der nach dem Trocknen und Entfernen des Methylenchlorids verbleibende Festkörper wird aus Methylenchlorid/Petrolether umkristallisiert. Man erhält 1-(4-Chlor-3-nitrophenoxy)-2-propanon vom Smp. 92-94°.

Ein Gemisch aus 23,8 g (0,104 Mol) 1-(4-Chlor-3-nitrophenoxy)-2-propanon, 2 g Raney-Nickel und 240 ml Dioxan, wird bei Normaldruck und Raumtemperatur hydriert. Nach 20 Stunden (Wasserstoffaufnahme: 103%) wird über Celite genutscht, mit Dioxan nachgewaschen und im Vakuum eingedampft. Chromatographische Reinigung des Rohprodukts mit Petrolether/Ether an Kieselgel und anschliessende Umkristallisation aus Ether/Petrolether ergibt 1-(3-Amino-4-chlorphenoxy)-2-propanon vom Smp. 49-51°C.

Ein Gemisch aus 11,4 g (57 mMol) 1-(3-Amino-4-chlorphenoxy)-2-propanon, 10 ml konz. Salzsäure und 150 ml Ethanol wird während 14 Stunden am Rückfluss gekocht und anschliessend im Vakuum eingedampft. Der Rückstand wird mit Eiswasser versetzt, mit 3 N Natronlauge alkalisch gestellt und mit Ether extrahiert. Das nach dem Trocknen und Eindampfen der organischen Phase erhaltene Rohprodukt wird mit Petrolether/Methylenchlorid an Kieselgel chromatographiert. Umkristallisation der reinen Fraktionen aus Ether/Petrolether ergibt 6-Amino-5-chlor-3-methylbenzofuran vom Smp. 73-75°.

Beispiel 13: Ein Gemisch aus 7,5 g (46,5 mMol) 6-Amino-3,5-dimethylbenzofuran, 7,5 ml (58 mMol) 2,5-Dimethoxy-tetrahydrofuran und 150 ml Dioxan wird mit 5 ml 6 N Salzsäure versetzt und während 40 Minuten am Rückfluss gekocht. Anschliessend wird im Vakuum eingedampft, der Rückstand in Methylenchlorid aufgenommen und dreimal mit Wasser gewaschen. Das nach dem Trocknen und Eindampfen der organischen Phase erhaltene Rohprodukt wird mit Petrolether/Ether an Kieselgel chromatographiert. Anschliessende Destillation der reinen Fraktionen im Kugelrohr (120°/4 $\cdot 10^{-2}$ Torr) ergibt 3,5-Dimethyl-6-(pyrrol-1-yl)-benzofuran vom Smp. 43-45°C.

Das Ausgangsmaterial kann folgendermassen hergestellt werden:
Zu einer Suspension von 61,25 g (0,4 Mol) 4-Methyl-3-nitrophenol, 60,8 g (0,44 Mol) gemahlenem Kaliumcarbonat, 39,9 g (0,267 Mol) Natriumiodid und 900 ml Dimethylformamid werden bei Raumtemperatur unter Rühren während 30 Minuten 40,7 g (0,44 Mol) Chloraceton getropft. Nach weiteren 3 Stunden bei Raumtemperatur wird genutscht und der Rückstand mit Dimethylformamid nachgewaschen. Die organische Lösung wird im Vakuum eingedampft und das erhaltene Rohprodukt zwischen Methylenchlorid und Wasser verteilt. Der nach dem Trocknen und Entfernen des Methylenchlorids verbleibende Rückstand wird mit Petrolether/Methylenchlorid an Kieselgel chromatographiert. Nachfolgende Umkristallisation aus Methylenchlorid/Petrolether ergibt 1-(4-Methyl-3-nitrophenoxy)-2-propanon vom Smp. 84-86°C.

Ein Gemisch aus 96,2 g (0,46 Mol) 1-(4-Methyl-3-nitrophenoxy)-2-propanon, 10 g 5% Pd/C und 960 ml Dioxan wird während 6 Stunden bei Raumtemperatur hydriert. Anschliessend wird vom Katalysator abfiltriert und im Vakuum eingedampft. Der erhaltene Rückstand wird in 2,5 l Ethanol aufgenommen, mit 400 ml konz. Salzsäure versetzt und während 30 Stunden am Rückfluss gekocht. Das Ethanol wird im Vakuum entfernt, der Rückstand mit Eiswasser verdünnt, mit 6 N Natronlauge alkalisch gestellt und mit Ether ausgezogen. Das nach dem Trocknen und Eindampfen der organischen Phase erhaltene Rohprodukt wird mit Methylenchlorid/Aceton an Kieselgel chromatographiert. Umkristallisation der reinen Fraktionen aus Petrolether ergibt 6-Amino-3,5-dimethylbenzofuran vom Smp. 64-65°.

Beispiel 14: Ein Gemisch aus 22,8 g (0,141 Mol) 6-Amino-3,5-dimethylbenzofuran, 65,8 g (0,283 Mol) 2,2'-Dibrom-diethylether, 36,1 g (0,279 Mol) N-Ethyl-diisopropylamin und 210 ml Ethanol wird während 16 Stunden am Rückfluss gekocht. Der nach dem Entfernen des Ethanols im Vakuum erhaltene Rückstand wird in Methylenchlorid aufgenommen und zweimal mit verdünnter Natriumbicarbonatlösung gewaschen. Die organische Phase wird getrocknet, eingedampft und das Rohprodukt mit Petrolether/Ether an Kieselgel chromatographiert. Der Ueberschuss an 2,2'-Dibromdiethylether wird am Wasserstrahlvakuum über eine Vigreux-Kolonne entfernt und der Rückstand mittels Festkörperdestillation (110-115°/$4 \cdot 10^{-2}$ Torr) gereinigt. Man erhält 3,5-Dimethyl-6-morpholino-benzofuran vom Smp. 69-70°.

Beispiel 15: Ein Gemisch aus 8 g (49,6 mMol) 6-Amino-3,5-dimethylbenzofuran, 23 g (0,1 Mol) 1,5-Dibrompentan, 12,9 g (0,1 Mol) N-Ethyldiisopropylamin und 60 ml Ethanol wird während 18 Stunden am Rückfluss gekocht. Der nach dem Eindampfen im Vakuum erhaltene Rückstand wird in Methylenchlorid aufgenommen und zweimal mit verdünnter Natriumbicarbonatlösung gewaschen. Die organische Phase wird getrocknet, eingedampft und das Rohprodukt mit Petrolether/Methylenchlorid an Kieselgel chromatographiert. Durch Destillation der reinen Fraktionen im Kugel-

rohr ($130°/10^{-1}$ Torr) erhält man 3,5-Dimethyl-6-piperidino-benzofuran vom Smp. 64-66°.

Beispiel 16: Ein Gemisch aus 9,1 g (50 mMol) 6-Amino-5-chlor-3-methyl-benzofuran, 10 ml (78 mMol) 2,5-Dimethoxy-tetrahydrofuran und 200 ml Dioxan wird mit 7 ml 5 N Salzsäure versetzt und während 16 Stunden bei Raumtemperatur gerührt. Anschliessend wird im Vakuum eingedampft und der Rückstand mit Petrolether/Methylenchlorid an Kieselgel chromatographiert. Kristallisation des erhaltenen Produkts aus Petrolether ergibt 5-Chlor-3-methyl-6-(pyrrol-1-yl)-benzofuran vom Smp. 49-52°.

Beispiel 17: Ein Gemisch aus 10 g (43,2 mMol) 3,5-Dimethyl-6-morpholino-benzofuran, 2 g 5% Pd/C und 200 ml Essigsäure-ethylester wird bei Raumtemperatur und einem Druck von 4 bar hydriert. Zur Vervollständigung des Umsatzes werden während der Hydrierung noch viermal je 2 g 5% Pd/C zugesetzt. Nach insgesamt 58 Stunden wird vom Katalysator abfiltriert und im Vakuum eingedampft. Durch Reinigung des Rückstandes mittels Flash-Chromatographie (Kieselgel, Petrolether/Ether) und Destillation der reinen Fraktionen im Kugelrohr ($130-140°/6 \cdot 10^{-2}$ Torr) erhält man 3,5-Dimethyl-6-morpholino-2,3-dihydrobenzofuran vom Smp. 57-58°.

Beispiel 18: Ein Gemisch aus 8 g (37,9 mMol) 3,5-Dimethyl-6-(pyrrol-1-yl)-benzofuran, 1,6 g 5% Pd/C und 80 ml Dioxan wird bei 35° und einem Druck von 4 bar hydriert. Zur Vervollständigung der Umsetzung werden während der Hydrierung noch dreimal je 0,8 g 5% Pd/C zugesetzt. Nach insgesamt 100 Stunden wird vom Katalysator abfiltriert und im Vakuum eingedampft. Durch Chromatographie des Rückstands an Kieselgel (Petrolether/Methylenchlorid) und Destillation der reinen Fraktionen im Kugelrohr ($120°/2 \cdot 10^{-2}$ Torr) erhält man 3,5-Dimethyl-6-(pyrrol-1-yl)-2,3-dihydrobenzofuran als farbloses Oel.
$^1$H-NMR-Spektrum (250 MHz, $CDCl_3$, $\delta$ in ppm): 1,36 (d, J≈7 Hz, 3H); 2,11 (s, 3H); 3,57 (m, 1H); 4,12 (t, J≈8 Hz, 1H); 4,72 (t, J≈8 Hz, 1H); 6,29 (m, 2H); 6,69 (s, 1H); 6,75 (m, 2H); 7,04 (s, 1H).

Beispiel 19: Ein Gemisch aus 2,95 g (20 mMol) 6-Amino-5-methyl-benzofuran, 3,17 g (24 mMol) 2,5-Dimethoxy-tetrahydrofuran und 50 ml Dioxan wird mit 2 ml 6 N Salzsäure versetzt und während 40 Minuten am Rückfluss gekocht. Anschliessend wird im Vakuum eingeengt, der Rückstand in Methylenchlorid aufgenommen und die organische Phase dreimal mit Wasser gewaschen. Das nach dem Trocknen und Entfernen des Methylenchlorids verbleibende Rohprodukt wird mit Ether/Petrolether an Kieselgel chromatographiert. Anschliessende Destillation der reinen Fraktionen im Kugelrohr (105°, $5 \cdot 10^{-2}$ Torr) ergibt 5-Methyl-6-(pyrrol-1-yl)-benzofuran als blassgelbes Oel.
$^1$H-NMR-Spektrum (250 MHz, $CDCl_3$, $\delta$ in ppm): 2,23 (s, 3H); 6,32 (m, 2H); 6,74 (m, 1H); 6,80 (m, 2H); 7,42 (s, 1H); 7,48 (s, 1H); 7,65 (d, J ≈ 2Hz, 1H).

Das Ausgangsmaterial kann folgendermassen hergestellt werden:

Ein Gemisch aus 15,3 g (0,1 Mol) 4-Methyl-3-nitrophenol. 16,8 g (0,11 Mol) Chloracetaldehyd-diethylacetal, 20,7 g (0,15 Mol) gemahlenem Kaliumcarbonat, 4,5 g (30 mMol) Natriumiodid und 100 ml Dimethylformamid wird während 12 Stunden am Rückfluss gekocht. Anschliessend wird genutscht, der Rückstand mit Dimethylformamid nachgewaschen und die Lösung im Vakuum eingedampft. Durch Chromatographie des Rückstands an Kieselgel (Methylenchlorid) und anschliessende Destillation der reinen Fraktionen (105-110°/$6 \cdot 10^{-2}$ Torr) erhält man 2-(4-Methyl-3-nitrophenoxy)-acetaldehyd-diethylacetal als gelbliches Oel.

12,4 g (46,1 mMol) dieses Oels werden in 130 ml Tetrahydrofuran aufgenommen, mit 4 g Raney-Nickel versetzt und während 7,5 Stunden bei Raumtemperatur hydriert. Anschliessend wird vom Katalysator abfiltriert und eingedampft. Destillation des Rückstandes im Kugelrohr (130°/ $5 \cdot 10^{-2}$ Torr) ergibt 2-(3-Amino-4-methylphenoxy)-acetaldehyd-diethylacetal als blassgelbes Oel.

2,39 g (10 mMol) dieses Oels werden in 75 ml Ethanol aufgenommen, mit 12 ml konz. Salzsäure versetzt und während 3,5 Stunden am Rückfluss gekocht. Anschliessend wird das Ethanol im Vakuum entfernt, der Rückstand mit Eiswasser verdünnt, mit 2 N Natronlauge alkalisch gestellt und mit Ether extrahiert. Das nach dem Trocknen und Eindampfen der organischen Phase verbleibende Rohprodukt wird im Kugelrohr (130°/ $6 \cdot 10^{-2}$ Torr) destilliert. Das Destillat wird mit Methylenchlorid an Kieselgel chromatographiert. Durch Destillation der reinen Säulenfraktionen im Kugelrohr (120°/$6 \cdot 10^{-2}$ Torr) erhält man 6-Amino-5-methylbenzofuran vom Smp. 74-76°.

Beispiel 20: In analoger Weise wie in einem der Beispiele 1-19 beschrieben, erhält man 5-Methyl-6-(pyrrol-1-yl)-2,3-dihydrobenzofuran.

1. Formulierungsbeispiel:

Tabletten, enthaltend 25 mg Wirkstoff, z.B. 3,5-Dimethyl-6-(pyrrol-1-yl)-benzofuran können folgendermassen hergestellt werden:

| Bestandteile (für 1000 Tabletten): | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

2. Formulierungsbeispiel:

Kautabletten, enthaltend 30 mg Wirkstoff, z.B. 3,5-Dimethyl-6-(pyrrol-1-yl)-benzofuran, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccarin | 1,0 g |
| 5%ige Gelatinelösung | q.s. |

Herstellung: Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

3. Formulierungsbeispiel:

Tabletten enthaltend 100 mg Wirkstoff, z.B. 3,5-Dimethyl-6-(pyrrol-1-yl)-benzofuran können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |

| | |
|---|---|
| Polyethylenglykol 6000 | 5,0 g |
| Talkum | 15,0 g |
| Magnesiumstearat | 4,0 g |
| entmineralisiertes Wasser | q,s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Patentansprüche für die Vertragsstaaten: BE, CH, LI, DE, FR, IT, LU, NL, SE

1. Benzofurane bzw. 2,3-Dihydro-benzofurane der Formel

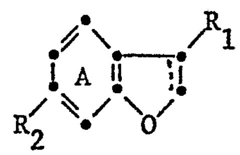

(I),

worin

$R_1$ für Wasserstoff oder einen aliphatischen Rest steht,

$R_2$ eine durch einen zweiwertigen, gegebenenfalls durch mindestens ein Heteroatom unterbrochenen aliphatischen Rest disubstituierte Aminogruppe bedeutet und der aromatische Ring

A zusätzlich substituiert sein kann, und ihre Salze.

2. Verbindungen gemäss Patentanspruch 1 der Formel I, worin $R_1$ für Wasserstoff, Niederalkyl, Niederalkenyl oder Niederalkinyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils im Niederalkylen- bzw. Niederalkenylenteil durch mindestens ein Aza, Niederalkylaza, Oxa oder Thia unterbrochenes Niederalkylenamino bzw. Niederalkenylenamino mit einer Doppelbindung bedeutet, und der aromatische Ring A zusätzlich durch Niederalkyl, Hydroxyniederalkyl, Halogenniederalkyl, Niederalkenyl, zwei benachbarte C-Atome überbrückendes Alkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze.

3. Verbindungen gemäss Patentanspruch 1 der Formel I, worin $R_1$ für Wasserstoff oder Niederalkyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils mit 3 bis und mit 7 Ringgliedern, jeweils im Niederalkylen- bzw. Niederalkenylenteil durch ein Aza, Niederalkylaza, Oxa oder Thia unterbrochenes Niederalkylenamino mit 5 oder 6 Ringgliedern bzw. Niederalkenylenamino mit einer Doppelbindung und 5 Ringgliedern bedeutet und der aromatische Ring A zusätzlich durch Niederalkyl, Hydroxyniederalkyl, Halogenniederalkyl, Niederalkenyl, zwei benachbarte C-Atome überbrückendes Niederalkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze.

4. Verbindungen gemäss Patentanspruch 1 der Formel

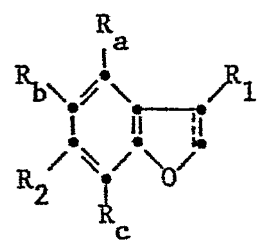

(Ia),

worin $R_1$ Wasserstoff oder Niederalkyl bedeutet, $R_2$ jeweils 5- bis 8-gliedriges Niederalkylenamino, Niederalkenylenamino, Azaniederalkylenamino, N'-Niederalkylaza-niederalkylenamino, Azaniederalkenylenamino, N'-Niederalkylaza-niederalkenylenamino, Oxa- bzw. Thia-niederalkylenamino, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, Hydroxyniederalkyl, Halogenniederalkyl, Niederalkenyl, Niederalkoxy, Niederalkylthio, Niederalkansulfinyl, Niederalkansulfonyl, Hydroxy, Halogen, Niederalkanoyloxy, Niederalkanoyl oder Nitro bedeuten oder $R_a$ gemeinsam mit $R_b$ 3- oder 4-gliedriges Alkylen

darstellen und $R_c$ die vorstehend für $R_c$ angegebenen Bedeutungen hat, und ihre Salze.

5. Verbindungen gemäss Patentanspruch 1 der Formel Ia, worin $R_1$ Wasserstoff oder Niederalkyl bedeutet, $R_2$ Niederalkylenamino mit 5 oder 6 Ringgliedern und 4 bis und mit 10 C-Atomen, Niederalkenylenamino mit einer oder zwei Doppelbindungen und 5 oder 6 Ringgliedern und 4 bis und mit 10 C-Atomen oder 4-Oxaniederalkylenamino mit 6 Ringgliedern und 4 bis und mit 10 C-Atomen, darstellt und $R_a$ und $R_c$ jeweils Wasserstoff bedeuten und $R_b$ Wasserstoff, Niederalkyl, Hydroxyniederalkyl, Halogenniederalkyl, Niederalkoxy, Niederalkylthio, Hydroxy, Halogen oder Niederalkanoyloxy darstellt oder $R_a$ und $R_b$ gemeinsam Niederalkylen mit 3 oder 4 Kettengliedern, z.B. mit 3 oder 4 C-Atomen, und $R_c$ Wasserstoff ist, und ihre Salze.

6. Verbindungen gemäss Patentanspruch 1 der Formel Ia, worin $R_1$ Wasserstoff oder Niederalkyl mit bis und mit 4 C-Atomen bedeutet, $R_2$ 1-Pyrrolyl, 4-Morpholinyl, 3-Pyrrolin-1-yl oder unverzweigtes 4- oder 6-gliedriges Alkylenamino darstellt, $R_a$ und $R_c$ jeweils Wasserstoff bedeuten und $R_b$ Wasserstoff, Niederalkyl mit bis und mit 4 C-Atomen oder Halogen bis und mit Atomnummer 35 darstellt oder $R_c$ Wasserstoff bedeutet und $R_a$ und $R_b$ gemeinsam 3 bis 4-gliedriges Alkylen oder einer der Reste $R_a$ und $R_b$ Halogen bis und mit Atomnummer 35 und der andere Niederalkyl mit bis 4 C-Atomen darstellen, und ihre Salze.

7. Verbindungen gemäss Patentanspruch 1 der Formel

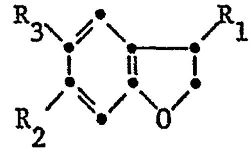

(Ib),

worin jeweils $R_1$ Wasserstoff oder Niederalkyl mit bis und mit 4 C-Atomen bedeutet, $R_2$ Pyrrolidin-1-yl, 1-Piperidyl, Pyrrol-1-yl

oder Morpholin-4-yl darstellt und $R_3$ Wasserstoff, Niederalkyl mit bis und mit 4 C-Atomen oder Halogen der Atomnummer bis und mit 35 bedeutet, und ihre Salze.

8. Verbindungen gemäss Anspruch 1 der Formel

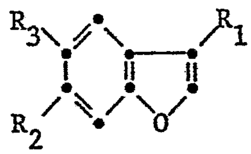

(Ic),

worin jeweils $R_1$ Wasserstoff oder Niederalkyl mit bis und mit 4 C-Atomen bedeutet, $R_2$ Pyrrolidin-1-yl, 1-Piperidyl, Pyrrol-1-yl oder Morpholin-4-yl darstellt und $R_3$ Wasserstoff, Niederalkyl mit bis und mit 4 C-Atomen oder Halogen der Atomnummer bis und mit 35 bedeutet, und ihre Salze.

9. Verbindungen gemäss Anspruch 1 der Formel Ic, worin $R_1$ Wasserstoff oder Niederalkyl mit bis und mit 4 C-Atomen bedeutet, $R_2$ Pyrrol-1-yl darstellt und $R_3$ Niederalkyl mit bis und mit 4 C-Atomen bedeutet.

10. 5-Chlor-6-(piperidin-1-yl)-2,3-dihydrobenzofuran oder ein Salz davon.

11. 5-Chlor-3-methyl-6-(piperidin-1-yl)-benzofuran oder ein Salz davon.

12. 5-Chlor-6-(piperidin-1-yl)-benzofuran oder ein Salz davon.

13. 5-Chlor-3-methyl-6-(pyrrolidin-1-yl)-benzofuran oder ein Salz davon.

14. 3,5-Dimethyl-6-(pyrrol-1-yl)-benzofuran.

15. 3,5-Dimethyl-6-morpholino-benzofuran oder ein Salz davon.

16. 5-Chlor-3-methyl-6-(pyrrol-1-yl)-benzofuran.

17. 3,5-Dimethyl-6-morpholino-2,3-dihydrobenzofuran oder ein Salz davon.

18. 3,5-Dimethyl-6-(pyrrol-1-yl)-2,3-dihydrobenzofuran.

19. 5-Methyl-6-(pyrrol-1-yl)-benzofuran.

20. 5-Methyl-6-(pyrrol-1-yl)-2,3-dihydrobenzofuran.

21. Verbindungen gemäss einem der Patentansprüche 1-20 mit antiinflammatorischer und/oder analgetischer Wirkung.

22. Verbindungen gemäss einem der Patentansprüche 1-21 als antiinflammatorisches und/oder analgetisches Mittel.

23. Verbindungen gemäss einem der Patentansprüche 1-21 zur Anwendung in einem Verfahren zur therapeutischen Behandlung des menschlichen oder tierischen Körpers.

24. Verbindungen gemäss einem der Patentansprüche 1-22 zur Verwendung gemäss Patentanspruch 23 als antiinflammatorischens und/oder analgetisches Mittel.

25. Pharmazeutische Präparate, enthaltend mindestens eine Verbindung gemäss einem der Patentansprüche 1-24.

26. Verwendung gemäss einem der Patentansprüche 1-24 zur Behandlung entzündlicher Erkrankungen und/oder von Schmerzzuständen.

27. Verwendung von Verbindungen gemäss einem der Patentansprüche 1-24 zur Herstellung pharmazeutischer Präparate.

28. Verfahren zur Herstellung von Verbindungen gemäss Patentanspruch 1, dadurch gekennzeichnet, dass man

(a) in einer Verbindung der Formel

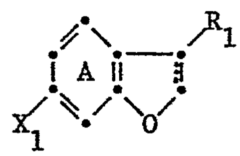

(II),

worin $X_1$ einen in $R_2$ überführbaren Rest bzw. durch $R_2$ ersetzbaren Rest bedeutet, $X_1$ in $R_2$ überführt bzw. durch $R_2$ ersetzt, oder,

(b) zur Herstellung von 2,3-Dihydrobenzofuranen der Formel I, in einer Verbindung der Formel

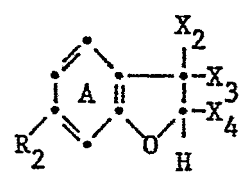

(III)

worin $X_2$ $R_1$ ist, einer der Reste $X_3$ und $X_4$ für einen durch Wasserstoff ersetzbaren Rest steht und der andere Wasserstoff ist bzw. $X_3$ und $X_4$ gemeinsam eine zusätzliche Bindung darstellen, $X_3$ oder $X_4$ durch Wasserstoff ersetzt bzw. die zusätzliche, durch $X_3$ und $X_4$ dargestellte Bindung reduziert, oder, wenn $R_1$ Wasserstoff ist, $X_2$ und $X_3$ gemeinsam für eine zweiwertige, durch 2 Wasserstoffatome ersetzbare Gruppe stehen und $X_4$ Wasserstoff ist, $X_2$ und $X_3$ jeweils durch 1 Wasserstoffatom ersetzt, oder,

(c) zur Herstellung von Benzofuranen bzw. 2,3-Dihydrobenzofuranen der Formel I, worin $R_1$ für einen aliphatischen Rest steht, in einer Verbindung der Formel

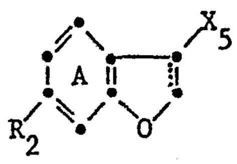

(IVa)

durch Umsetzung mit einer Verbindung der Formel $R_1-X_5'$ (IVb), worin einer der Reste $X_5$ und $X_5'$ gegebenenfalls reaktionsfähiges verestertes oder verethertes Hydroxy bedeutet und der andere für einen metallischen Rest steht oder worin $X_5$ und $X_5'$ jeweils Halogen bedeuten, und unter Abspaltung von $X_5-X_5'$ den Rest $R_1$ einführt, oder

(d) eine Verbindung der Formel

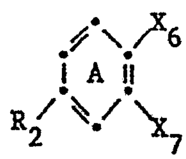

(V),

worin $X_6$ für die Gruppe der Formel $-CH(R_1)-CH_2-X_6'$ steht, wobei $X_6'$. für einen abspaltbaren Rest steht, und $X_7$ Hydroxy ist, oder worin, falls $R_1$ der Formel I für einen aliphatischen Rest steht, $X_6$ die Diazoniumgruppe oder Halogen bedeutet und $X_7$ für die Gruppe der Formel $-O-CH_2-CH = R_1'$ steht, wobei $R_1'$ ein dem Rest $R_1$ entsprechender, doppelt gebundener aliphatischer Rest ist, zu einer 2,3-Dihydrobenzofuranverbindung der Formel I cyclisiert bzw. eine Verbindung der Formel V, worin $X_6$ Wasserstoff ist und $X_7$ die Gruppe der Formel $-O-CH_2-C(=O)-R_1$ oder eine derivatisierte Form davon steht, oder worin $X_6$ die Gruppe der Formel $-C(R_1)=CH-X_6''$ darstellt, wobei $X_6''$ für einen abspaltbaren Rest steht, und $X_7$ Hydroxy ist, zu einer Benzofuranverbindung der Formel I cyclisiert, oder,

(e) zur Herstellung von Benzofuranen der Formel I, in einer Verbindung der Formel

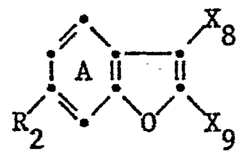

(V),

worin einer der Reste $X_8$ und $X_9$ für einen durch Wasserstoff ersetzbaren Rest steht und der andere Wasserstoff oder, im Fall von $X_8$, ein von Wasserstoff verschiedener Rest $R_1$ bedeutet, $X_8$ bzw. $X_9$ durch Wasserstoff ersetzt, oder,

(f) zur Herstellung von Benzofuranen der Formel I, aus einer der Verbindung der Formel

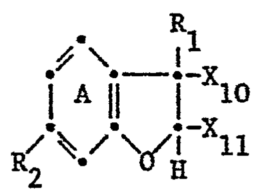

(VII),

worin einer der Reste $X_{10}$ und $X_{11}$ für einen abspaltbaren Rest steht und der andere Wasserstoff ist, oder worin $X_{10}$ und $X_{11}$ jeweils Halogen, Carboxy oder Wasserstoff sind, unter Bildung einer zusätzlichen Bindung, eine Verbindung $X_{10} - X_{11}$ abspaltet, oder,

(g) zur Herstellung von Benzofuranden der Formel I, worin $R_1$ für einen aliphatischen Rest der Formel

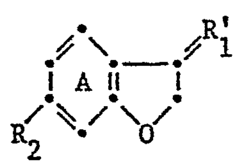

(VIII),

worin $R_1'$ ein dem Rest $R_1$ entsprechender, von Wasserstoff verschiedener, doppelt gebundener aliphatischer Rest darstellt, isomerisiert und, wenn erwünscht, eine erhaltene erfindungsgemässe Verbindung in eine andere erfindungsgemässe Verbindung überführt und/oder gewünschtenfalls ein erhaltenes Salz in die freie Verbindung überführt und/oder gewünschtenfalls eine erhaltene erfindungsgemässe Verbindung

mit salzbildenden Eigenschaften in ein Salz überführt und/oder gewünschtenfalls ein erhaltenes Isomerengemisch in die einzelnen Isomeren auftrennt.

29. Verfahren nach Anspruch 28, dadurch gekennzeichnet, dass man eine Verbindung der Formel

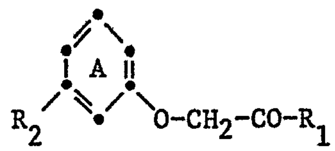

mit Hilfe eines Cyclisierungsmittels, wie eines Titan(III)halogenids, z.B. Titan(III)chlorid, in einem, insbesondere inerten Lösungsmittel, wie einem Niederalkanol, gegebenenfalls unter Kühlen oder Erwärmen, zu einer Verbindung der Formel

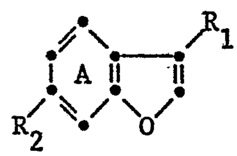

(I')

cyclisiert und/oder eine Verbindung der Formel (I') durch Reduktion, wie mit Hilfe eines Reduktionsmittels, z.B. eines gegebenenfalls komplexen Hydrides, wie mit einem Alkalimetallborhydrid, z.B. Kaliumborhydrid, in einem Lösungsmittel, wie einem Ether, z.B. Dioxan, gegebenenfalls unter Kühlen in eine Verbindung der

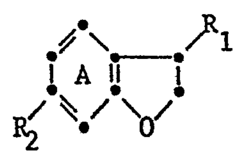

(I'')

überführt und/oder, wenn erwünscht, ein verfahrensgemäss erhältliches Salz in die freie Verbindung oder in ein anderes Salz überführt, eine verfahrensgemäss erhältiche freie Verbindung, in analoger Weise für Verbindungen der Formel (I), in eine andere freie Verbindung oder in ein Salz überführt und/oder gewünschtenfalls ein verfahrensgemäss erhältliches Isomerengemisch in seine Komponenten auftrennt.

30. Die in dem Verfahren gemäss einem der Patentansprüche 28 und 29 verwendeten neuen Ausgangsstoffe und/oder Zwischenprodukte.

31. Die nach den Verfahren gemäss einem der Ansprüche 28 und 29 erhältlichen Verbindungen.

FO 7.4 DH/jt*/hc*

Patentansprüche für den Vertragsstaat AT

1. Verfahren zur Herstellung von Benzofuranen bzw. 2,3-Dihydrobenzofuranen der Formel

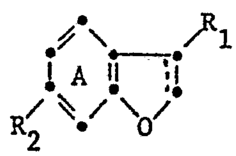
(I),

worin

$R_1$ für Wasserstoff oder einen aliphatischen Rest steht,

$R_2$ eine durch einen zweiwertigen, gegebenenfalls durch mindestens ein Heteroatom unterbrochenen aliphatischen Rest disubstituierte Aminogruppe bedeutet und der aromatische Ring

A zusätzlich substituiert sein kann, und ihrer Salze, dadurch gekennzeichnet, dass man

(a) in einer Verbindung der Formel

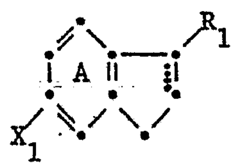
(II),

worin $X_1$ einen in $R_2$ überführbaren Rest bzw. durch $R_2$ ersetzbaren Rest bedeutet, $X_1$ in $R_2$ überführt bzw. durch $R_2$ ersetzt, oder,

(b) zur Herstellung von 2,3-Dihydrobenzofuranen der Formel I, in einer Verbindung der Formel

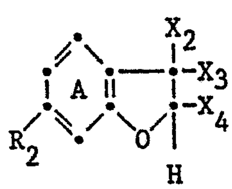
(III),

worin $X_2$ $R_1$ ist, einer der Reste $X_3$ und $X_4$ für einen durch Wasserstoff ersetzbaren Rest steht und der andere Wasserstoff ist bzw. $X_3$ und $X_4$ gemeinsam eine zusätzliche Bindung darstellen, $X_3$ oder $X_4$ durch Wasserstoff ersetzt bzw. die zusätzliche, durch $X_3$ und $X_4$ dargestellte Bindung reduziert, oder, wenn $R_1$ Wasserstoff ist, $X_2$ und $X_3$ gemeinsam für eine zweiwertige, durch 2 Wasserstoffatome ersetzbare Gruppe stehen und $X_4$ Wasserstoff ist, $X_2$ und $X_3$ jeweils durch 1 Wasserstoffatom ersetzt, oder,

(c) zur Herstellung von Benzofuranen bzw. 2,3-Dihydrobenzofuranen der Formel I, worin $R_1$ für einen aliphatischen Rest steht, in eine Verbindung der Formel

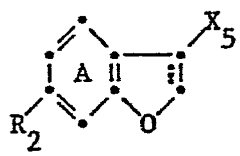

(IVa)

durch Umsetzung mit einer Verbindung der Formel $R_1$-$X_5$' (IVb), worin einer der Reste $X_5$ und $X_5$' gegebenenfalls reaktionsfähiges verestertes oder verethertes Hydroxy bedeutet und der andere für einen metallischen Rest steht oder worin $X_5$ und $X_5$' jeweils Halogen bedeuten, und unter Abspaltung $X_5$-$X_5$' den Rest $R_1$ einführt, oder

(d) eine Verbindung der Formel

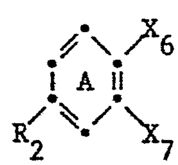

worin $X_6$ für die Gruppe der Formel $-CH(R_1)-CH_2-X_6'$ wobei $X_6'$ für einen abspaltbaren Rest steht, und $X_7$ Hydroxy ist, oder worin, falls $R_1$ der Formel I für einen aliphatischen Rest steht, $X_6$ die Diazoniumgruppe oder Halogen bedeutet und $X_7$ für die Gruppe

der Formel $-O-CH_2-CH = R_1'$ steht, wobei $R_1'$ ein dem Rest $R_1$ entsprechender, doppelt gebundener aliphatischer Rest ist, zu einer 2,3-Dihydrobenzofuranverbindung der Formel I cyclisiert bzw. eine Verbindung der Formel V, worin $X_6$ Wasserstoff ist und $X_7$ die Gruppe der Formel $-O-CH_2-C(=O)-R_1$ oder eine derivatisierte Form davon steht, oder worin $X_6$ die Gruppe der Formel $-C(R_1)=CH-X_6''$ darstellt, wobei $X_6''$ für einen abspaltbaren Rest steht, und $X_7$ Hydroxy ist, zu einer Benzofuranverbindung der Formel I cyclisiert, oder,

(e) zur Herstellung von Benzofuranen der Formel I, in einer Verbindung der Formel

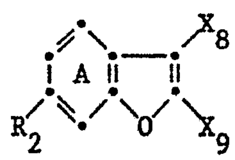

(VI),

worin einer der Reste $X_8$ und $X_9$ für einen durch Wasserstoff ersetzbaren Rest steht und der andere Wasserstoff oder, im Fall von $X_8$, ein von Wasserstoff verschiedener Rest $R_1$ bedeutet, $X_8$ bzw. $X_9$ durch Wasserstoff ersetzt, oder,

(f) zur Herstellung von Benzofuranen der Formel I, aus einer der Verbindung der Formel

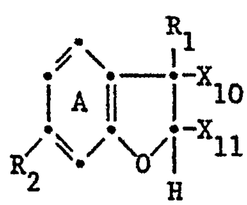

(VII),

worin einer der Reste $X_{10}$ und $X_{11}$ für einen abspaltbaren Rest steht und der andere Wasserstoff ist, oder worin $X_{10}$ und $X_{11}$ jeweils Wasserstoff sind, unter Bildung einer zusätzlichen Bindung, eine Verbindung $X_{10} - X_{11}$ abspaltet, oder,

(g) zur Herstellung von Benzofuranen der Formel I, worin $R_1$ für einen aliphatischen Rest steht, eine Verbindung der Formel

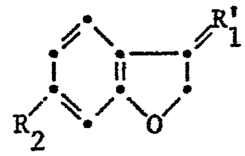

(VIII),

worin $R_1'$ ein dem Rest $R_1$ entsprechender, von Wasserstoff verschiedener, doppelt gebundener aliphatischer Rest darstellt, isomerisiert und, wenn erwünscht, eine erhaltene erfindungsgemässe Verbindung in eine andere erfindungsgemässe Verbindung überführt und/oder gewünschtenfalls ein erhaltenes Salz in die freie Verbindung überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Isomeren auftrennt.

2. Verfahren nach Anspruch 1, dadurch gekennzeichnet, dass man eine Verbindung der Formel

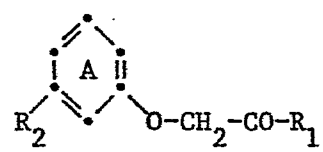

mit Hilfe eines Cyclisierungsmittels, wie eines Titan(III)halogenids, z.B. Titan(III)chlorid, in einem, insbesondere inerten Lösungsmittel wie einem Niederalkanol, gegebenenfalls unter Kühlen oder Erwärmen zu einer Verbindung der Formel

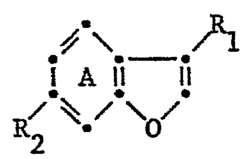

(I')

cyclisiert und/oder eine Verbindung der Formel (I') durch Reduktion wie mit Hilfe eines Reduktionsmittels, z.B. eines gegebenenfalls komplexen Hydrides, wie mit einem Alkalimetallborhydrid, z.B. Kaliumborhydrid, in einem Lösungsmittel, wie einem Ether, z.B. Dioxan, gegebenenfalls unter Kühlen in einer Verbindung der Formel

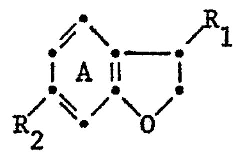

(I")

überführt und/oder, wenn erwünscht, ein verfahrensgemäss erhältliches Salz in die freie Verbindung oder in ein anderes Salz überführt, eine verfahrensgemäss erhältliche freie Verbindung, in analoger Weise für Verbindungen der Formel (I), in eine andere freie Verbindung oder in ein Salz überführt und/oder gewünschtenfalls ein verfahrensgemäss erhältliches Isomerengemisch in seine Komponenten auftrennt.

3. Verfahren nach Anspruch 1 oder 2, dadurch gekennzeichnet, dass man Verbindungen der Formel I, worin $R_1$ für Wasserstoff, Niederalkyl, Niederalkenyl oder Niederalkinyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils im Niederalkylen- bzw. Niederalkenylenteil durch mindestens ein Aza, Niederalkylaza, Oxa oder Thia unterbrochenes Niederalkylenamino bzw. Niederalkenylenamino mit einer Doppelbindung bedeutet, und der aromatische Ring A zusätzlich durch Niederalkyl, Hydroxyniederalkyl, Halogenniederalkyl, Niederalkenyl, zwei benachbarte C-Atome überbrückendes Alkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze herstellt.

4. Verfahren nach Anspruch 1 oder 2, dadurch gekennzeichnet, dass man Verbindungen der Formel I, worin $R_1$ für Wasserstoff oder Niederalkyl steht, $R_2$ Niederalkylenamino, welches zusätzlich ein oder zwei ortho-anellierte Benzosysteme aufweisen kann, Niederalkenylenamino mit einer oder zwei Doppelbindungen, wobei Niederalkenylenamino mit einer Doppelbindung zusätzlich ein ortho-anelliertes Benzosystem aufweisen kann, jeweils mit 3 bis und mit 7 Ringgliedern, jeweils im Niederalkylen- bzw. Niederalkenylenteil durch ein Aza, Niederalkylaza, Oxa oder Thia unterbrochenes Niederalkylenamino mit 5 oder 6 Ringgliedern bzw. Niederalkenylenamino mit einer Doppelbindung und 5 Ringgliedern bedeutet und der aromatische Ring A zusätzlich durch Niederalkyl, Hydroxyniederalkyl, Halogenniederalkyl, Niederalkenyl, zwei benachbarte C-Atome überbrückendes Niederalkylen mit 3 oder 4 Kettengliedern, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_2$, unsubstituiert ist, und ihre Salze herstellt.

5. Verfahren nach Anspruch 1 oder 2, dadurch gekennzeichnet, dass man Verbindungen der Formel

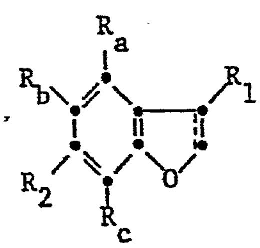

(Ia),

worin $R_1$ Wasserstoff oder Niederalkyl bedeutet, $R_2$ jeweils 5- bis 8-gliedriges Niederalkylenamino, Niederalkenylenamino, Azaniederalkylenamino, N'-Niederalkylaza-niederalkylenamino, Azaniederalkenylenamino, N'-Niederalkylaza-niederalkenylenamino, Oxa- bzw. Thia-niederalkylenamino, Isoindol-2-yl, Isoindolin-2-yl, Indolin-1-yl oder Indol-1-yl darstellt und $R_a$, $R_b$ sowie $R_c$ unabhängig voneinander Wasserstoff, Niederalkyl, Hydroxyniederalkyl, Halogenniederalkyl, Niederalkenyl, Niederalkoxy, Niederalkylthio, Niederalkansulfinyl, Niederalkansulfonyl, Hydroxy, Halogen, Niederalkanoyloxy, Niederalkanoyl oder Nitro bedeuten oder $R_a$ gemeinsam mit $R_b$ 3- oder 4-gliedriges Alkylen darstellen und $R_c$ die vorstehend für $R_c$ angegebenen Bedeutungen hat und ihre Salze herstellt.

6. Verfahren nach Anspruch 1 oder 2, dadurch gekennzeichnet, dass man Verbindungen der Formel Ia, worin $R_1$ Wasserstoff oder Niederalkyl bedeutet, $R_2$ Niederalkylenamino mit 5 oder 6 Ringgliedern und 4 bis und mit 10 C-Atomen oder 4-Oxaniederalkylenamino mit 6 Ringgliedern und 4 bis und mit 10 C-Atomen, darstellt und $R_a$ und $R_c$ jeweils Wasserstoff bedeuten und $R_b$ Wasserstoff, Niederalkyl, Hydroxyniederalkyl, Halogenniederalkyl, Niederalkoxy, Niederalkythio, Hydroxy, Halogen oder Niederalkanoyloxy darstellt oder $R_a$ und $R_b$ gemeinsam Niederalkylen mit 3 oder 4 Kettengliedern, z.B. mit 3 oder 4 C-Atomen und $R_c$ Wasserstoff ist, und ihre Salze herstellt.

7. Verfahren nach Anspruch 2, dadurch gekennzeichnet, dass man Verbindungen der Formel Ia, worin $R_1$ Wasserstoff oder Niederalkyl mit bis und mit 4 C-Atomen bedeutet, $R_2$ 1-Pyrrolyl, 4-Morpholinyl, 3-Pyrrolin-1-yl oder unverzweigtes 4- oder 6-gliedriges Alkylenamino darstellt, $R_a$ und $R_c$ jeweils Wasserstoff bedeuten und $R_b$ Wasserstoff, Niederalkyl mit bis und mit 4 C-Atomen oder Halogen bis und mit Atomnummer 35 darstellt oder $R_c$ Wasserstoff bedeutet und $R_a$ und $R_b$ gemeinsam 3 bis 4-gliedriges Alkylen oder einer der Reste $R_a$ und $R_b$ Halogen bis und mit Atomnummer 35 und der andere Niederalkyl mit bis 4 C-Atomen darstellen, und ihre Salze herstellt.

8. Verfahren nach Anspruch 1 oder 2, dadurch gekennzeichnet, dass man Verbindungen der Formel

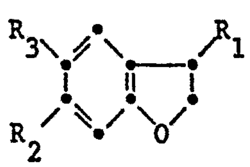

(Ib),

worin jeweils $R_1$ Wasserstoff oder Niederalkyl mit bis und mit 4 C-Atomen bedeutet, $R_2$ Pyrrolidin-1-yl, 1-piperidyl, Pyrrol-1-yl oder Morpholin-4-yl darstellt und $R_3$ Wasserstoff, Niederalkyl mit bis und mit 4 C-Atomen oder Halogen der Atomnummer bis und mit 35 bedeutet, und ihre Salze herstellt.

9. Verfahren nach Anspruch 1 oder 2, dadurch gekennzeichnet, dass man Verbindungen der Formel

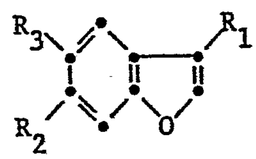

(Ic),

worin jeweils $R_1$ Wasserstoff oder Niederalkyl mit bis und mit 4 C-Atomen bedeutet, $R_2$ Pyrrolidin-1-yl, 1-Piperidyl, Pyrrol-1-yl oder Morpholin-4-yl darstellt und $R_3$ Wasserstoff, Niederalkyl mit bis und mit 4 C-Atomen oder Halogen der Atomnummer bis und mit 35 bedeutet, und ihre Salze herstellt.

10. Verfahren nach Anspruch 1 oder 2, dadurch gekennzeichnet, dass man Verbindungen der Formel Ic, worin $R_1$ Wasserstoff oder Niederalkyl mit bis und mit 4 C-Atomen bedeutet, $R_2$ Pyrrol-1-yl darstellt und $R_3$ Niederalkyl mit bis und mit 4 C-Atomen bedeutet, herstellt.

11. Verfahren nach Anspruch 1 oder 2, dadurch gekennzeichnet, dass man 5-Chlor-6-(piperidin-1-yl)-2,3-dihydrobenzofuran oder ein Salz davon herstellt.

12. Verfahren nach Anspruch 1 oder 2, dadurch gekennzeichnet, dass man 5-Chlor-3-methyl-6-(piperidin-1-yl)-benzofuran oder ein Salz davon herstellt.

13. Verfahren nach Anspruch 1 oder 2, dadurch gekennzeichnet, dass man 5-Chlor-6-(piperidin-1-yl)-benzofuran oder ein Salz davon herstellt.

14. Verfahren nach Anspruch 1 oder 2, dadurch gekennzeichnet, dass man 5-Chlor-3-methyl-6-(pyrrolidin-1-yl)-benzofuran oder ein Salz davon herstellt.

15. Verfahren nach Anspruch 1 oder 2, dadurch gekennzeichnet, dass man 3,5-Dimethyl-6-(pyrrol-1-yl)-benzofuran herstellt.

16. Verfahren nach Anspruch 1 oder 2, dadurch gekennzeichnet, dass man 3,5-Dimethyl-6-morpholino-benzofuran oder ein Salz davon herstellt.

17. Verfahren nach Anspruch 1 oder 2, dadurch gekennzeichnet, dass man 5-Chlor-3-methyl-6-(pyrrol-1-yl)-benzofuran herstellt.

18. Verfahren nach Anspruch 1 oder 2, dadurch gekennzeichnet, dass man 3,5-Dimethyl-6-morpholino-2,3-dihydrobenzofuran oder ein Salz davon herstellt.

19. Verfahren nach Anspruch 1 oder 2, dadurch gekennzeichnet, dass man 3,5-Dimethyl-6-(pyrrol-1-yl)-2,3-dihydrobenzofuran herstellt.

20. Verfahren nach Anspruch 1 oder 2, dadurch gekennzeichnet, dass man 5-Methyl-6-(pyrrol-1-yl)-benzofuran herstellt.

21. Verfahren nach Anspruch 1 oder 2, dadurch gekennzeichnet, dass man 5-Methyl-6-(pyrrol-1-yl)-2,3-dihydrobenzofuran herstellt.

22. Verfahren zur Herstellung pharmazeutischer Präparate, dadurch gekennzeichnet, dass man eine Verbindung gemäss einem der Patentansprüche 1-21, gegebenenfalls unter Beimischung von üblichen Hilfs- und Trägerstoffen, zu pharmazeutischen Präparaten verarbeitet.

FO 7.4 DH/jt*/hc*

0106800

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 83 81 0449

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | EP - A - 0 006 524 (CIBA-GEIGY)<br>* Das ganzes Dokument *<br>-- | 1-25, 27-29 | C 07 D 307/79<br>C 07 D 405/04<br>//C 07 D 307/83<br>C 07 D 295/08<br>C 07 D 295/10<br>C 07 C 49/84<br>C 07 C 93/14 |
| Y | US - A - 4 143 055 (E. OCCELLI)<br>* Das ganzes Dokument *<br>---- | 1-25, 27-29 | |
| | | | RECHERCHIERTE SACHGEBIETE (Int. Cl. ³)<br>C 07 D 307/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-25,27-29
Unvollständig recherchierte Patentansprüche:
Nicht recherchierte Patentansprüche: 26: Verfahren zur therapeutischen Behandlung des menschlichen oder tierischen Körpers.
Grund für die Beschränkung der Recherche:

30,31: Unklarheit.

| Recherchenort | Abschlußdatum der Recherche | Prüfer |
|---|---|---|
| Den Haag | 02-01-1984 | ALLARD |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82